# EUROPÄISCHE PATENTSCHRIFT

(11) **EP 0 658 199 B1**
(45) Veröffentlichungstag und Bekanntmachung des Hinweises auf die Patenterteilung: **21.10.1998**
(21) Anmeldenummer: 93919177.1
(22) Anmeldetag: 26.08.1993
(51) Int. Cl.: C12N 15/12, C12N 15/63, C12N 15/67, C12N 15/85, C07K 14/49

(54) **HERSTELLUNG VON HETERODIMEREM PDGF-AB MIT HILFE EINES BICISTRONISCHEN VEKTORSYSTEMS IN SÄUGERZELLEN**
PRODUCTION OF HETERODIMER PDGF-AB BY MEANS OF A BICISTRONIC VECTOR SYSTEM IN MAMMALIAN CELLS
PRODUCTION DE PDGF-AB HETERODIMERES A L'AIDE D'UN SYSTEME VECTORIEL BICISTRONIQUE DANS DES CELLULES MAMMIFERES

(30) Priorität: 27.08.1992 DE 4228457
(43) Veröffentlichungstag der Anmeldung: 21.06.1995
(73) Patentinhaber: Beiersdorf Aktiengesellschaft, 20245 Hamburg (DE); Gesellschaft für Biotechnologische Forschung mbH (GBF), D-38124 Braunschweig (DE)
(72) Erfinder: EICHNER, Wolfram, D-22301 Hamburg (DE); ACHTERBERG, Volker, D-20257 Hamburg (DE); DÖRSCHNER, Albrecht, D-20357 Hamburg (DE); MEYER-INGOLD, Wolfgang, D-22457 Hamburg (DE); MIELKE, Heiko, D-21629 Neu Wulmstorf (DE); DIRKS, Wilhelm, D-38106 Braunschweig (DE); WIRTH, Manfred, D-38300 Wolfenbüttel (DE); HAUSER, Hansjörg, D-38104 Braunschweig (DE)
(74) Vertreter: UEXKÜLL & STOLBERG
(86) Internationale Anmeldenummer: EP9302295
(87) Internationale Veröffentlichungsnummer: WO9405786

(56) Entgegenhaltungen:
- EP-A- 0 259 632
- WO-A-90/01550
- WO-A-90/08163
- WO-A-93/03143
- JOURNAL OF BIOLOGICAL CHEMISTRY, vol. 263, no. 31, 05 November 1988, American Society for Molecular Biology Inc., Baltimore, MD (US); A. OSTMAN et al., pp. 16202-16208
- EUROPEAN JOURNAL OF BIOCHEMISTRY, vol. 185, no. 1, October 1989, Springer Verlag, Berlin (DE); W. EICHNER et al., pp. 135-140

## Beschreibung

Die Erfindung betrifft die rekombinante Herstellung von PDGF-AB (rPDGF-AB) in Säugerzellen als Wirtszellen, welches im wesentlichen frei ist von den homodimeren Begleitprodukten PDGF-AA und -BB .

Seit vielen Jahren ist es möglich, einzelne Proteine, deren Gene durch Klonierung isoliert wurden, nach Manipulation und Gentransfer in verschiedenen prokaryontischen und eukaryontischen Zellen herzustellen. Für das Erreichen der vollen biologischen Aktivität vieler Proteine sind korrekte Faltungen, richtiges Prozessieren und gegebenenfalls auch posttranslationale Modifikationen erforderlich, welche in prokaryontischen und niederen eukaryontischen Expressionssystemen oftmals nicht korrekt ausgeführt werden. Aus diesem Grund bedient man sich häufig Säugerzellen als Wirte. Säugerzellen sind darüber hinaus in der Lage, große Mengen von Proteinen zu sekretieren.

Aus verschiedenen Gründen bedarf es oftmals der simultanen Herstellung zweier oder mehrerer Proteinketten. Zum Beispiel sind viele natürliche Proteine in ihrer funktionellen Form aus mehreren Untereinheiten zusammengesetzt (z.B. Antikörper). Natürlicherweise erfolgt die Assoziation verschiedener Untereinheiten von komplexen Proteinen nach der Proteinsynthese. An dieser Assoziation sind häufig andere Komponenten des zellulären Apparates als Katalysatoren oder Kontrollelemente beteiligt, wobei es gelegentlich auch zu Umfaltungen der ursprünglichen Strukturen kommt. Störungen der Assoziation, z.B. durch ungleiche Synthese der einzelnen Komponenten, können sowohl für die zu bildenden Proteine als auch für die Wirtszelle negative Konsequenzen haben. Natürlicherweise unterliegt dieses System einer ausgefeilten, meist zellspezifischen Regulation. Da diese Regulation in genetisch manipulierten Zellen im allgemeinen nicht nachstellbar ist, wurden die nachfolgend erläuterten Alternativen zur simultanen Herstellung mehrerer Fremdproteine entwickelt und angewandt:
1) Die Gene werden getrennt in Expressionsvektoren integriert und dann in einem geeigneten Verhältnis in die Zellen co-transferiert. Dies setzt voraus, daß mehrere Plasmidkopien gleichzeitig stabil aufgenommen und bei der Teilung weiter-getragen werden. Das Verhältnis der Expression der verschiedenen Gene zueinander hängt sowohl von der Kopienzahl als auch von der Integrationsstelle im Genom der Wirtszelle ab. Durch aufwendige Screeningverfahren ist es möglich, Zellklone zu isolieren, welche die einzelnen Genprodukte im gewünschten Verhältnis zueinander exprimieren.
2) Um die Kopienzahl zu nivellieren, werden die verschiedenen Gene in unabhängigen Transkriptionseinheiten auf einem Vektor plaziert. Dies sichert weitgehend die stöchiometrische Repräsentanz der Gene, aber auch dieses Verfahren ist mit Problemen behaftet. Selbst wenn nämlich Expressionseinheiten mit Promotoren gleicher Stärke verwendet werden, ist keineswegs sichergestellt, daß die mRNAs, welche für die verschiedenen Proteine kodieren, die gleiche Stabilität und Translationseffizienz aufweisen. Auch die Transkriptionseffizienz beider Gene muß nicht zwangsläufig identisch sein. In diesem Fall wird mit Hilfe von gentechnischen Tricks (Positionierung der Transkriptionseinheiten zueinander, Modulation der Stärke der einzelnen Promotoren durch Wegnehmen oder Hinzufügen einzelner Elemente) die Stöchiometrie der Expression schrittweise hergestellt.
3) Zur Vermeidung der Probleme im Zusammenhang mit der Stabilität der mRNA verschiedener Transkripte wurden bi- oder multicistronische Vektoren entwickelt. Hierzu liegen die einzelnen Leseraster der die Proteinketten kodierenden Genabschnitte - Cistrons - auf einer Transkriptionseinheit (Expressionseinheit). Die Expression des multicistronischen Gens erfolgt durch einen einzigen Promotor. Bei derartigen Vektoren wird normalerweise das erste Cistron sehr effizient translatiert, die nachfolgenden aber in Abhängigkeit von den intercistronischen Sequenzen. Verwendet man für diese intercistronischen Sequenzen normale 5' nicht translatierte Sequenzen (5'UTR) aus monocistronischen Genen, so ist die Expression des nachfolgenden Cistrons meist sehr niedrig (in der Regel um 0,5 bis 2% der Translation des ersten Cistrons, Kaufman et al., 1987; Boel et al., 1987). Diese Effizienz konnte zunächst durch Einfügen von Leadersequenzen (High Efficiency Leadern, HEL) auf etwa 20% gesteigert werden. Mit der Entdeckung und Verwendung von bestimmten zellulären und viralen Sequenzen, welche eine interne Translationsinitiation ermöglichen (IRES; Jackson et al., 1990), war es möglich, ein Translationsverhältnis zwischen dem ersten und dem nachfolgenden Cistron von 3:1 zu erzielen.

Die Schlüsselrolle bei der Verwendung bi- oder multicistronischer Vektoren spielt die Translation. Normalerweise erfolgt die Initiation der Translation in Eukaryonten nach dem "cap"-abhängigen Mechanismus, im Verlaufe dessen ein Prä-Initiationskomplex aus Proteinen und RNA am 5'Ende einer mRNA mit "cap" (methyliertes Nukleotid) aufgebaut wird. Von dort aus wird ein geeignetes Translationsinitiationskodon ausgesucht, von dem ausgehend die Translation gestartet wird. Man glaubt, daß dies über einen "Scanning"-Prozeß abläuft, wobei sich der Prä-Initiationskomplex entlang der mRNA in 3'Richtung bewegt. Auf diese Weise wird, von einigen Ausnahmen abgesehen, immer das am 5'Ende liegende Cistron effizient translatiert (Kozak, 1989). Alle nachfolgenden Cistrons werden gar nicht oder sehr ineffizient translatiert. Die Translations-Effizienz der nachfolgenden Cistrons konnte durch Optimierung des Abstandes zwischen den Genen (intercistronische Regionen; Kozak, 1987; Wirth et al., 1991) oder durch Verwendung von sogenannten "high efficiency leader"-Sequenzen (HEL, s.o.) verbessert werden (z.B. Falcone und Andrews, 1991 und Ref. darin). HEL's sind solche 5'nicht translatierten Bereiche von Genen oder auch anderen Sequenzen, welche die Initiation der "cap"-abhängigen Translation stimulieren. Auch bei derartigen Konstrukten sind jedoch die erreichbaren Expressionswerte für das zweite und die nachfolgenden Cistrons immer deutlich geringer als die des "cap"-abhängig regulierten ersten Cistrons.

Ein in den letzten Jahren aufgedeckter Mechanismus zur internen Translationsinitiation benutzt spezifische Nukleinsäuresequenzen. Zu diesen Sequenzen zählen die nicht translatierten Bereiche einzelner Picorna-Viren, z.B. Polio-Virus, Encephalomyocarditis-Virus, (Pelletier und Sonenberg, 1988; Jang et al., 1988; Jang et al., 1989) sowie einiger zellulärer Proteine, z.B. BiP (Macejak und Sarnow, 1991). Bei den Picorna-Viren sorgt ein kurzer Abschnitt des 5'nicht translatierten Bereichs, das sogenannte IRES (internal ribosomal entry site), für die interne Bindung eines Prä-Initiationskomplexes. Darüber hinaus sind weitere Bereiche aus dieser Region für die effiziente Initiation dieser Translation notwendig. So zeigt es sich z.B., daß für eine effiziente Translation nicht nur die 400 Basenpaare stromaufwärts des IRES, sondern auch der extreme 5'Teil der Picorna-Virus nicht-translatierten Region notwendig ist (Simoes und Sarnow, 1991). Auf der anderen Seite führt das "capping", die Voraussetzung für den normalen Initiationsmechanismus der Translation, zu einer Reduktion der Effizienz der internen Initiation von Polio-Virus IRES, wenn er am 5'Ende einer entsprechenden mRNA lokalisiert ist (Hambidge und Sarnow, 1991). Der negative Effekt wird aufgehoben, wenn die IRES für die Initiation des zweiten Cistrons verantwortlich ist, also zwischen "cap" und IRES ein Cistron liegt.

IRES-Elemente können also als Initiatoren für die effiziente Translation von Leserastern fungieren. Dabei beeinflussen sie die "cap"-abhängige Translation des ersten Cistrons nicht. Auch umgekehrt scheint eine Beeinflussung der IRES-abhängigen Initiation unabhängig von der "cap"-abhängigen Translationsinitiation zu sein. Die Mechanismen beider Vorgänge unterscheiden sich auch deutlich in der Verwendung verschiedener zellulärer Faktoren (Meerovitch et al., 1989; Jang und Wimmer, 1990). In der vergangenen Zeit wurden mehrere Untersuchungen publiziert, bei denen bicistronische Expressionsplasmide verwendet wurden (Adam et al., 1991; Ghattas et al., 1991; Kaufman et al.,1991; Wood et al., 1991; Wirth et al., 1991). Da aber offensichtlich die "cap"-abhängige Translation stärker ist als die IRES-abhängige Translation, konnte eine stöchiometrische Expression zweier Proteinketten nicht erreicht werden. Die bisherigen Verwendungen konzentierten sich deshalb auf die Verwendung von Selektionsmarkern im zweiten Cistron. Die enge Expressionskoppelung des Selektionsmarkers mit dem zu exprimierenden Gen, welches das erste Cistron darstellt, ist besonders vorteilhaft bei der Selektion auf Hochexpression, insbesondere, wenn eine vorausgehende Genamplifikation erforderlich ist.

Die Synthese äquimolarer Proteinmengen von bi- oder multicistronischen Expressionsvektoren ist jedoch bisher nicht erreicht worden. Die äquimolare Expression zweier verschiedener Proteinketten ist für die rekombinante Herstellung des Wachstumsfaktors aus Blutplättchen, "Platelet-Derived-Growth-Factor" (PDGF), einem der Hauptmitogene im menschlichen Blutserum, von besonderer Bedeutung. Aus menschlichen Thrombozyten aufgereinigtes PDGF besteht aus zwei unterschiedlichen, aber nahe verwandten Polypeptidketten, die durch Disulfidbrücken miteinander verknüpft sind. Unter reduzierenden Bedingungen zerfällt das dimere PDGF in seine monomeren Untereinheiten, wovon die größere (Mᵣ 15-17.000 D) als PDGF-A-Kette und die kleinere (Mᵣ 14.000 D) als PDGF-B-Kette bezeichnet wurde (Johnsson et al., 1984).

Die Proteinketten PDGF-A und -B werden von verschiedenen Genen kodiert. Die vollständige Struktur beider Genprodukte konnte durch cDNA-Klonierung aufgeklärt werden (Ratner et al., 1985, Betsholtz et al., 1986). Dabei zeigte es sich, daß beide PDGF-Moleküle zunächst als ungewöhnlich lange Vorläufermoleküle, sog. Precursoren, synthetisiert und anschließend intrazellulär zu den reifen PDGF-Ketten prozessiert werden. Durch alternatives Splicen lassen sich zwei verschiedene PDGF-A-Transkripte erklären, die sich durch An- oder Abwesenheit eines 69-bp Segmentes im 3'-Bereich unterscheiden (Betsholtz et al., 1986; Wise et al., 1989). Durch dieses Insert kommt es zu einer Änderung im codierenden Abschnitt mit der Folge, daß eine kurze (PDGF-A_{K}, 110 Aminosäuren) und eine lange (PDGF-A_{L}, 125 Aminosäuren) Form der PDGF-A-Kette gebildet wird. Beide Varianten sind als normale zelluläre Proteine nebeneinander nachweisbar, wobei die kürzere Form die häufigere Spezies ist (Matoskova et al., 1989; Young et al., 1990).

Beide Gene sind auf unterschiedlichen Chromosomen lokalisiert und zeigen einen hohen Homologiegrad. Eine Vielzahl von Arbeiten zeigt, daß beide Gene unterschiedlichen Regulationsmechanismen unterliegen. Eine Folge davon ist, daß beide PDGF-Ketten natürlicherweise in verschiedenen Zelltypen in unterschiedlichem Verhältnis zueinander produziert werden.

Alle drei möglichen Isoformen des PDGF (AA, AB und BB) kommen natürlicherweise vor und sind in Thrombozyten in sogenannten α-Granula gespeichert. Aus überlagerten menschlichen Blutplättchen kann neben dem die Hauptmenge bildenden PDGF-AB Heterodimer auch PDGF-BB zu etwa 30 % isoliert werden (Hammacher et al., 1988). Frisch präparierte Blutplättchen enthalten auch einen hohen Anteil (27%) an PDGF-AA (Hart et al., 1990). Es kann daher angenommen werden, daß in den Vorläuferzellen der Thrombozyten, den Megakaryozyten, der Anteil beider Homodimere zusammen etwa dem AB-Heterodimer-Anteil entspricht. Da die Konzentration jeder PDGF-Spezies im Thrombozyten direkt korrelieren sollte mit ihrer individuellen Bedeutung im Wundheilungsgeschehen, kommt insbesondere der häufigsten Isoform, dem PDGF-AB, eine herausragende Bedeutung auf der Suche nach einem "Wundheilungshormon" zu.

Jede der verschiedenen Isoformen besitzt biologische Aktivität *in vitro.* Erst die Verfügbarkeit der hochreinen, rekombinanten PDGF-Isoformen (Hoppe et al., 1989; Hoppe et al., 1990) machte vergleichende Studien zur Differenzierung der unterschiedlichen Wirkungsspektren der verschiedenen PDGF-Spezies möglich. Inzwischen belegen eine Reihe von Untersuchungen die unterschiedliche Potenz von PDGF-AA, AB und BB im Chemotaxis- und DNA-Proliferationstest (Hosang et al., 1989; Nister et al., 1988; Reilly & Broski, 1989; Siegbahn et al., 1990), sowie deren unterschiedlichen Einfluß auf die Freisetzung von Inositol-1,4,5-trisphosphat, Produktion von Diacylglycerol und [Ca²⁺]ᵢ-Mobilisierung (Block et al., 1989; Sachinidis et al., 1990 A, 1990 B). Zwei unterschiedliche PDGF-Rezeptorpopulationen, von denen der PDGF-α-Rezeptor alle PDGF-Isoformen und der β-Rezeptor nur PDGF-BB bindet (Hart et al., 1988; Heldin et al., 1988) liefern eine plausible Erklärung dafür, wie sich Wirkungsunterschiede der PDGF-Isoformen über deren unterschiedliche Potenz zur Rezeptoraktivierung entfalten können. Die meßbaren unterschiedlichen *in vitro*-Effekte der PDGF-Isoformen, zusammen mit dem Nachweis zweier verschiedenener Rezeptorpopulationen, lassen Rückschlüsse auf unterschiedliche *in vivo* Wirkungsspektren von PDGF-AA, AB und BB zu. Daher ist die Produktion von reinem PDGF-AB, ohne PDGF-BB oder PDGF-AA als Begleitproteine, wünschenswert. Um ein homogenes, gut charakterisiertes Heterodimer zu erhalten, müßten die Homodimere sonst durch Reinigung vollständig eliminiert werden, was durch die sehr ähnlichen chromatographischen Eigenschaften aller PDGF Spezies zusätzlich erschwert wird.

Eine Reihe verschiedener Wege zur Herstellung von rekombinanten PDGF-Homodimeren, insbesondere PDGF-BB, sind zum Teil schon seit längerer Zeit bekannt (Kelly et al., 1985; Heldin et al., 1986; Hoppe et al., 1989; Beckmann et al., 1988; Bywater et al., 1988; Stroobant & Waterfield 1984). Ein Herstellungsverfahren für hochreines PDGF-AB wurde von Hoppe et al. (1990, s.a. PCT/EP 90/00 063) beschrieben. Hier werden die getrennt in unterschiedlichen E.coli-Zellen hergestellten, inaktiven Monomere durch in vitro-Renaturierung in biologisch aktives PDGF-AB überführt.

Die bislang synthetisierten Genprodukte der drei PDGF-Isoformen weisen, trotz variierender Länge der A- bzw. B-Einzelstränge, weitgehend übereinstimmende biologische Aktivität auf.

Für die heterologe Expression von PDGF-AB Heterodimeren in eukaryontischen Systemen gelten die eingangs erwähnten Kriterien der simultanen Expression zweier (oder mehrerer) Proteine. Die bisher publizierten Strategien zur Herstellung von PDGF-AB in rekombinanten CHO-Zellen (Östman et al., 1988) und mit Hilfe von Hefe-Expressionssystemen [EP 0 259 632] entsprechen dem oben unter 2) erläuterten Fallbeispiel, wo sich beide PDGF-Gene in unabhängigen Transkriptionseinheiten auf einem Vektor befinden. Die Quantifizierung der auf diese Weise in CHO-Zellen exprimierten unterschiedlichen PDGF-Dimere ergab 19% für PDGF-AA, 69% für PDGF-AB und 12% für PDGF-BB (Östman et al., 1988).

Eine Grundvoraussetzung für die bevorzugte Synthese von PDGF-AB Heterodimeren mit Hilfe eukaryontischer Expressionssysteme ist daher nicht nur in der stöchiometrischen Repräsentanz beider Gene, sondern in erster Linie auch in deren koordinierter Expression zu sehen. Daher bieten sich bicistronische Expressionseinheiten als mögliche Hilfsmittel für die Expression heterodimerer Proteine und damit des PDGF-AB an. In WO 90/01550 wird ein derartiges System auch für die Expression von PDGF beschrieben. Wie unter Punkt 3) oben näher erläutert, liefern diese Konstrukte jedoch nur sehr limitierte Expressionsraten für das zweite (und nachfolgende) Cistron. Abhängig von der im ersten Cistron lokalisierten PDGF-Kette werden vorwiegend Homodimere dieses Typs gebildet. Bisher in der Literatur beschriebene Versuche, beide PDGF-Gene mit Hilfe anderer Expressionssysteme in einer eukaryontischen Zelle zu exprimieren, führten zu Homodimer-Nebenproduktanteilen im Bereich von 30% oder mehr. Um dennoch mit diesen Zellsystemen PDGF-AB zu erhalten, müssen aufwendige und extrem verlustreiche Reinigungstechniken angewendet werden.

Aufgabe der Erfindung ist es daher, ein Expressionssystem zur Synthese äquimolarer Proteinmengen von bicistronischen Expressionsvektoren zu schaffen, mit Hilfe dessen PDGF-AB ohne wesentliche Verunreinigung durch die jeweiligen Homopolymere hergestellt werden kann.

Erfindungsgemäß konnte die Aufgabe durch die Verwendung eines Konstrukts gelöst werden, welches in an sich bekannter Weise von der IRES-Sequenz zwischen dem ersten und zweiten Cistron Gebrauch macht, jedoch das für PDGF-B kodierende Gen in das erste Cistron einbringt. Überraschenderweise hat es sich erfindungsgemäß gezeigt, daß die Wirtszellen nach Transfektion mit diesen Konstrukten PDGF-AB ohne nennenswerten Anteil an Homodimeren sezernieren. Ausbeute und Wirtschaftlichkeit der nachgeschalteten Proteinreinigungsverfahren wird dadurch beträchtlich verbessert.

Gegenstand der Erfindung ist demgemäß eine bicistronische Expressionseinheit zur rekombinanten Herstellung von heterodimerem PDGF-AB in Säugerzellen als Wirtszellen, welche gekennzeichnet ist durch die allgemeine Formel

p - 5'UTR - C₁ - IRES - C₂ - 3'UTR - polyA,

in der
p ein transkriptionaler Promotor ist,
5'UTR eine nicht translatierte Nukleotidsequenz ist,
C₁ ein Cistron ist, welches ein für die B-Kette von PDGF, ein biologisch aktives Analogon oder ein Fragment derselben kodierendes Gen enthält,
IRES eine Nukleotidsequenz viralen, zellulären oder synthetischen Ursprungs ist, die in der Stufe der Translation für die interne Initiation verantwortlich ist,
C₂ ein Cistron ist, welches ein für die A-Kette von PDGF oder ein biologisch aktives Analogon oder ein Fragment derselben kodierendes Gen enthält,
3'UTR eine nicht translatierte Nukleotidsequenz,
und polyA ein Polyadenylierungssignal ist,
wobei C₁, IRES und C₂ operativ miteinander verknüpft sind.

Als Promotoren kommen alle diejenigen Promotoren in Betracht, die in eukaryontischen Zellen wirksam sind, d. h., die die Genexpression in eukaryontischen Zellen initiieren können. Insbesondere können alle konstitutiven und induzierbaren Promotoren viralen (beispielweise die "Long terminal repeats, LTR's, von Retroviren oder Herpes Simplex Thymidin-Kinase Promotor), zellulären (beispielsweise Interferon- oder Ubiquitin-Promotor) oder synthetischen Ursprungs verwendet werden. Erfindungsgemäß ist der SV40-Promotor bevorzugt.

Die 5'UTR und die 3'UTR sind beliebige, in der Regel nichttranslatierte Nukleotidsequenzen, die regulierende Elemente enthalten können. Erfindungsgemäß geeignet sind beispielsweise die Sequenzen aus SV-40 nach Artelt et al. (1988).

Das erste Cistron, C₁, kann die vollständige PDGF-B Vorläufersequenz (SEQ ID Nr. 3), deren Analoga und jegliches Fragment enthalten, welches für eine biologisch wirksame PDFG-B Kette kodiert. Insbesondere kommen in diesem Zusammenhang das zur PDGF-B-Kette homologe *v-sis-Gen* (Produkt des Simian Sarcoma Virus (SSV)) sowie die Basenpaare 283 bis 609 gemäß SEQ ID Nr. 3 in Betracht, welche die reife PDFG-B Kette kodieren.

Analog kann das zweite Cistron, C₂, die lange oder kurze PDGF-A Vorläufersequenz (PDGF-A_{L} oder PDGF-A_{K} - SEQ ID Nr. 1) sowie jegliche Fragmente enthalten, welche für eine biologisch aktive PDGF-A Kette kodieren. In Betracht kommt insbesondere das Fragment gemäß den Basenpaaren 353 bis 682 gemäß SEQ ID Nr. 1, welches für die reife PDGF-A Kette kodiert.

Als IRES können alle diejenigen Sequenzen viralen, zellulären oder synthetischen Ursprungs verwendet werden, welche eine interne Bindung der Ribosomen vermitteln. Beispiele für derartige Sequenzen sind die IRES aus Poliovirus Typ 1 gemäß SEQ ID Nr. 5, welche die ersten 628 Nukleotide der 5' nicht-translatierten Region des Poliovirus Typ 1 einschließt, sowie ferner die 5'UTR des Enzephalomyocarditis Virus (EMV), des "Theilers murine encephalomyelitis virus" (TMEV), des "foot and mouth disease virus" (FMDV), des "bovine enterovirus" (BEV), des "coxsackie B virus" (CBV), des "human rhinovirus" (HRV) und die "human immunoglobulin heavy chain binding protein" (BIP) 5'UTR, die Drosophila Antennapediae 5'UTR, die Drosophila Ultrabithorax 5'UTR oder genetische Hybride oder Fragmente aus den oben angeführten Sequenzen. Erfindungsgemäß bevorzugt ist die IRES aus Poliovirus Typ 1 gemäß SEQ ID Nr. 5.

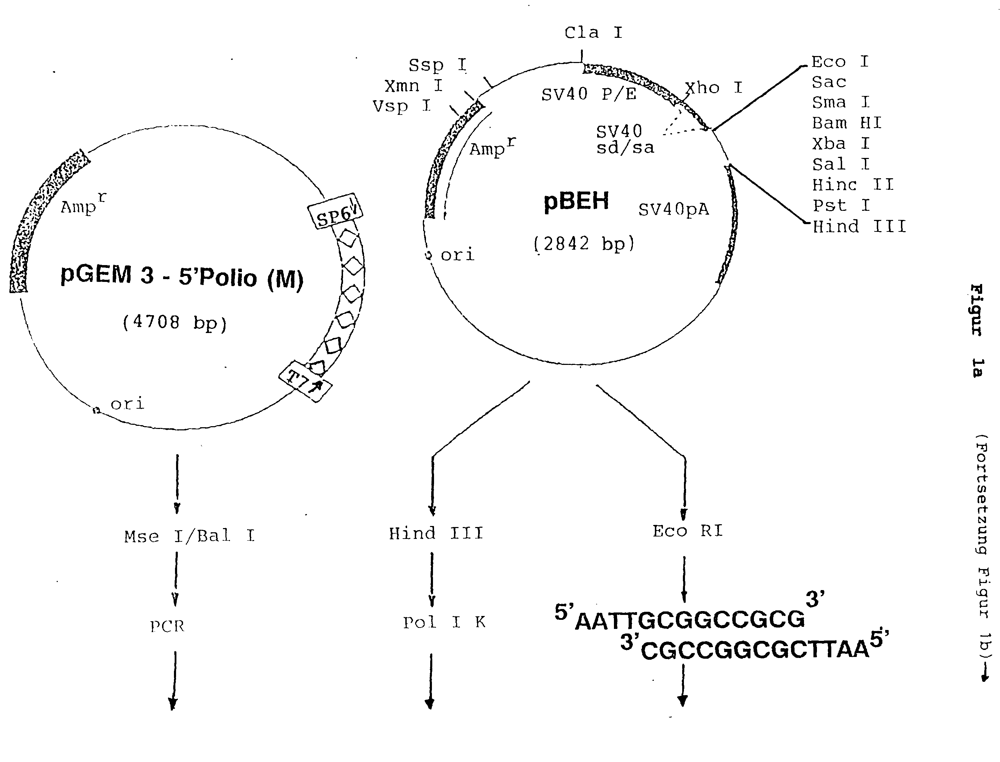

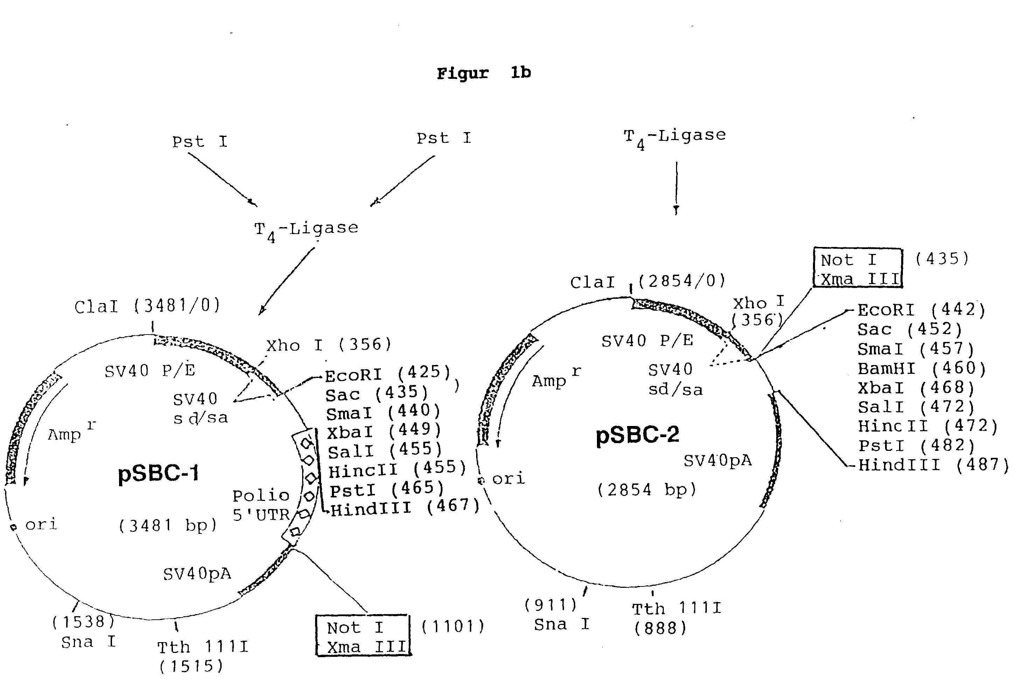

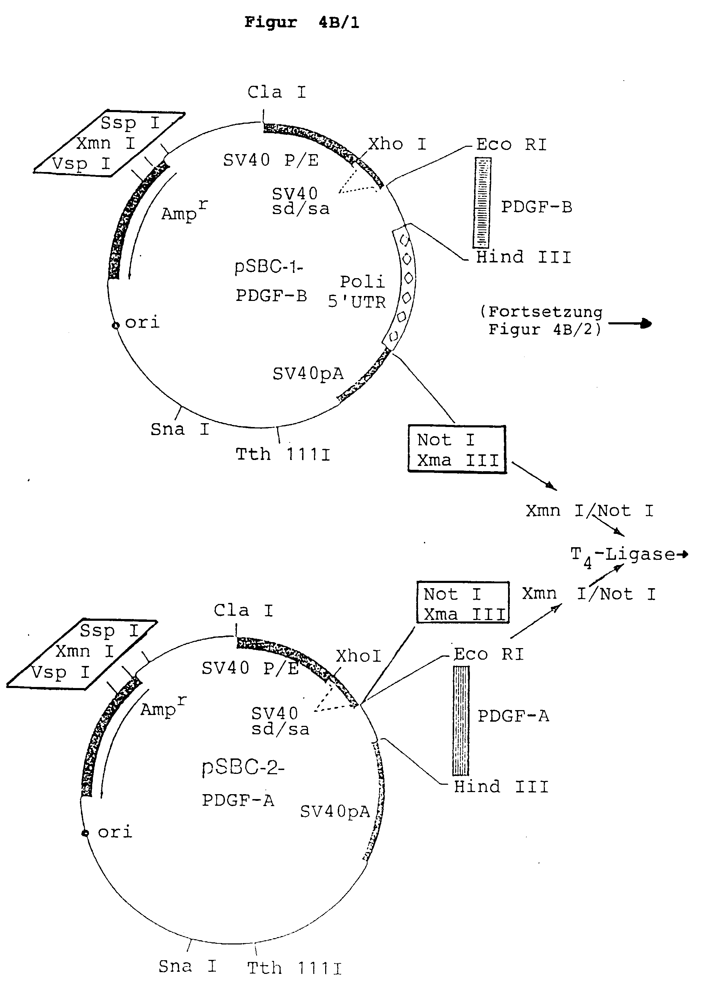

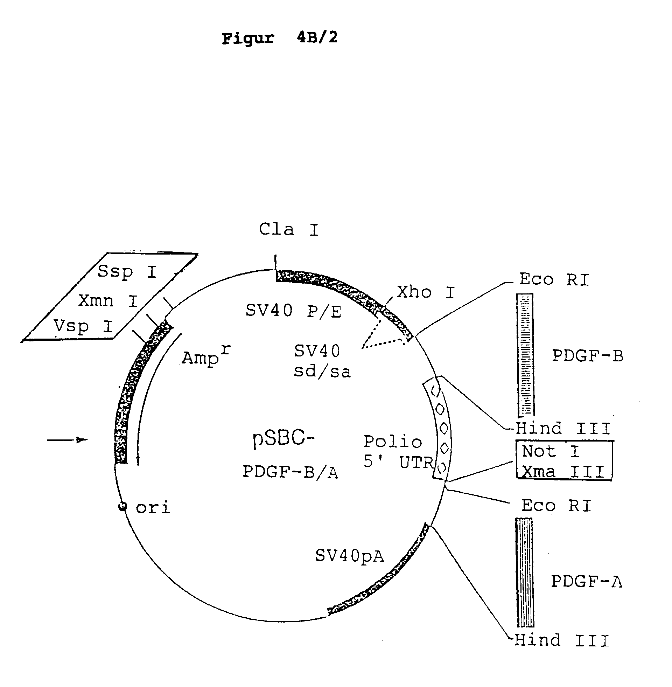

Gegenstand der Erfindung sind ferner rekombinante DNA Vektoren, welche die erfindungsgemäße Expressionseinheit enthalten. Ein erfindungsgemäß bevorzugter Vektor ist in Figur 4B und dessen Herstellung in den Figuren 1 bis 3 dargestellt.

Die Erfindung schließt ferner Wirtszellen ein, welche Säugerzellen sind und mit einem Vektor transformiert sind, der die erfindungsgemäße Expressionseinheit trägt. Vorzugsweise handelt es sich um CHO- oder BHK-Zellen, wobei letztere besonders bevorzugt sind. Eine erfindungsgemäß transformierte BHK-Zelle wurde unter der Bezeichnung 91-21-4D am 11. 8. 1992 bei der Deutschen Sammlung von Mikroorganismen und Zellkulturen GmbH (DSM) hinterlegt. Ihr wurde die Hinterlegungsnummer DSM ACC2045 zugeteilt.

Die Erfindung schließt darüber hinaus Verfahren zur Herstellung von heterodimerem rPDGF-AB ein, in deren Verlauf Säugerzellen als Wirtszellen, welche die erfindungsgemäße Expressionseinheit operativ insertiert enthalten, in einem geeigneten Medium kultiviert werden und das so erzeugte rPDGF-AB von den Zellen und dem Medium abgetrennt wird. Als Medium kommen alle bekannten Medien zum Kultivieren von Säugerzellen in Betracht, einschließlich synthetischer, proteinfreier oder proteinarmer Produktionsmedien. Erfindungsgemäß wurde DMEM (Dulbecco's Modified Eagle Medium), angereichert mit 4,5 g/l Glukose und 5 bis 10 % FCS, bevorzugt.

Das rPDGF-AB wird nach herkömmlichen Verfahren (vgl. beispielsweise Östmann et al. 1988) von den Zellen und dem Medium abgetrennt. Vorzugsweise wird erfindungsgemäß ein für PDGF-AA entwickeltes und hoch effizientes Verfahren (Eichner et al., 1989) angewendet.

Durch Kultivieren der oben beschriebenen, erfindungsgemäßen Wirtszellen ist schließlich heterodimeres rPDGF-AB erhältlich, welches im wesentlichen frei ist von homodimeren Begleitprodukten. Überraschenderweise hat es sich gezeigt, daß die mit dem erfindungsgemäßen Konstrukt transformierten Wirtszellen das heterodimere PDGF-AB mit einer Reinheit von 90% und mehr, bezogen auf die Gesamtmenge des gebildeten PDGF, sezernieren. Erfindungsgemäß bevorzugt ist PDGF-AB durch Kultivieren von BHK-Zellen, transformiert mit dem erfindungsgemäßen Konstrukt, beispielsweise derjenigen Zellen, welche unter der Bezeichnung 91-21-4D am 11. 8. 1992 bei der Deutschen Sammlung von Mikroorganismen und Zellkulturen GmbH (DSM) hinterlegt wurden (Hinterlegungsnummer DSM ACC2045), zugänglich.

Das erfindungsgemäß erhältliche rPDGF-AB unterscheidet sich von den bisher bekannten rekombinanten PDGF-AB Produkten in erster Linie durch seinen hohen Reinheitsgrad.

Den im Stand der Technik bekannten Produkten haften, abhängig von deren Herstellung, in mehrfacher Hinsicht Nachteile an. So ist es bekannt, daß die heterologe Genexpression in Hefezellen, wie in EP 259 632 oder 288 307 beschrieben, zu Proteinprodukten mit gegenüber dem humanen Produkt veränderten Glykosylierungsmustern führt. Zudem ist in Hefezellen exprimiertes PDGF-B zumindest teilweise unvollständig prozessiert und/oder proteolytisch abgebaut (vgl. WO 92/01716). Derartige Produkte weisen somit ein verändertes Kohlenhydratmuster auf und sie sind mit proteolytischen Abbauprodukten verunreinigt. Zur Vermeidung der vorgenannten Nachteile beschreibt die WO 92/01716 Verfahren zur Herstellung modifizierter PDGF-Ketten, bei denen die Konsensus-Sequenzen für die Glykosylierung bzw. die Protease sensitiven Domänen entfernt sind. Derartige Modifikationen beeinflussen jedoch die biologische Aktivität des Produktes (vgl. WO 92/01716).

Gemäß einer besonders bevorzugten Ausführungsform der Erfindung wird durch Kultivieren von erfindungsgemäß transformierten BHK-Zellen, und insbesondere durch Kultivieren der Wirtszellen 91-21-4D mit der Hinterlegungs-Nr.: DSM ACC2045 heterodimeres rPDGF-AB gewonnen.

Ferner ist aus der WO 90/08163 die rekombinante Herstellung von PDGF-AB in Bakterienzellen, insbesondere in E. *coli* bekannt, welche zwangläufig zu einem nicht glykosylierten Produkt führt. Eine nach diesem Verfahren in *E. coli* Zellen exprimierte PDGF-B-Kette ist jedoch aminoterminal um 12 Aminosäuren verkürzt. Darüberhinaus muß das Produkt aus Bakterien *in vitro* renaturiert werden, ein Vorgang, bei dem die korrekte inter- und intramolekulare Bildung der Disulphidbrücken und die korrekte Faltung des Proteins nicht gewährleistet ist mit der Folge, daß die immunologischen Eigenschaften des Produkts verändert und die biologische Aktivität beeinflußt werden können.

Das heterodimere rPDGF-AB gemäß der Erfindung wird vorzugsweise zusammen mit pharmazeutisch verträglichen Hilfs- und Trägerstoffen als pharmazeutisches Präparat, insbesondere für die Wundheilung formuliert. In diesem Zusammenhang kann es als Wirkstoff in Pflastern, Wundverbänden und dergleichen enthalten sein. Es ist besonders für die topische Verabreichung geeignet, es kommen jedoch auch Applikationen in Betracht, in deren Verlauf der Wirkstoff in die Wunde eingebracht oder subkutan verabreicht wird. Beispielsweise kann das PDGF-AB in einer geeigneten Matrix mit Depotfunktion im Wundrandbereich subkutan appliziert werden oder direkt subkutan injiziert werden.

Ferner eignet sich das erfindungsgemäße rPDGF-AB zur Herstellung von kosmetischen Präparaten, zum Beispiel zur Hautregeneration, zur Hautglättung, zur Verhinderung der Narbenbildung oder der Hautalterung sowie zur Anwendung bei Sonnenbrand.

Geeignete Hilfs- und Trägerstoffe schließen Cellulose-Gele auf wässriger Basis, biologisch abbaubare Polymere sowie jegliche Salben- und Cremebasis und Sprays ein. Ferner können zusätzliche Wirkstoffe, welche die Wundheilung beeinflussen, wie beispielsweise Kollagen, Fibronektin, Faktor XIII, Fibroblasten Wachstumsfaktor (aFGF, bFGF), Transformierender Wachstumsfaktor Typ α oder β, Epidermis Wachstumsfaktor, Insulin oder "Insulin-like Growth Factor" (IGF I, II) oder weitere Wachstumsfaktoren in den erfindungsgemäßen Präparaten enthalten sein. Die erfindungsgemäßen Produkte können beispielsweise auch in Wundverbänden in wässriger Lösung vorliegen.

Die Erfindung wird nachfolgend anhand von Beispielen erläutert:

### 1. Beschreibung der Figuren:

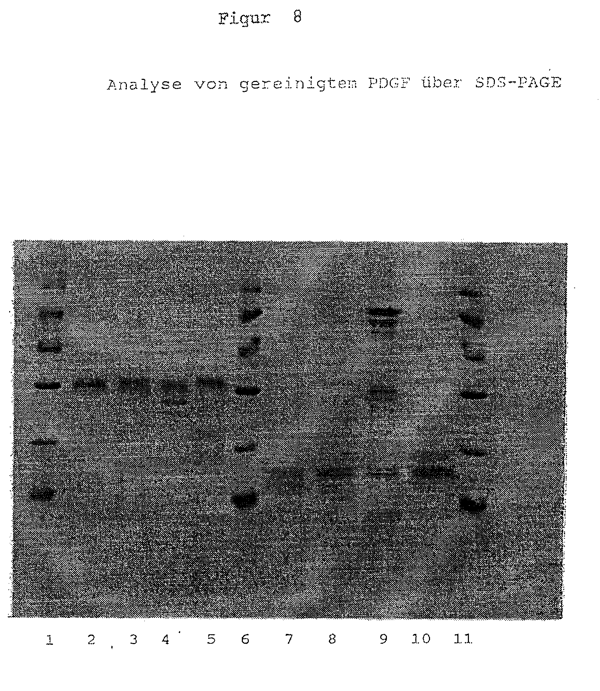

- Fig. 1): Schematische Darstellung der Herstellung der Basisvektoren pSBC-1 und pSBC-2.
- Fig. 2): Vektor M13BCL1; in der Vektorkarte ist der c-sis (PDGF-B) homologe Bereich aus pMVW-2 angegeben. Der Bereich des reifen PDGF-B und der NcoI/SalI (SEQ ID Nr. 8 + 9) Adapter sind durch schwarze Balken hervorgehoben.
- Fig. 3): Schematische Darstellung der Rekonstruktion der vollständigen PDGF-B-Vorläufersequenz.
- Fig. 4A +B): Schematische Darstellung der Konstruktion des bicistronischen Vergleichs-Vektors pSBC-A/B (Fig. 4A) und des erfindungsgemäßen Expressionsvektors pSBC-PDGF-B/A (Fig. 4B) aus den Basisvektoren pSBC-1 und pSBC-2. Die Expressionsvektoren pSBC-PDGF-A/B und pSBC-PDGF-B/A unterscheiden sich dabei durch die Orientierung der kodierenden cDNA-Sequenzen der PDGF-A- und -B-Kette, d.h. durch ihre Lokalisierung auf dem Plasmid als in Leserichtung erstes bzw. zweites Cistron.

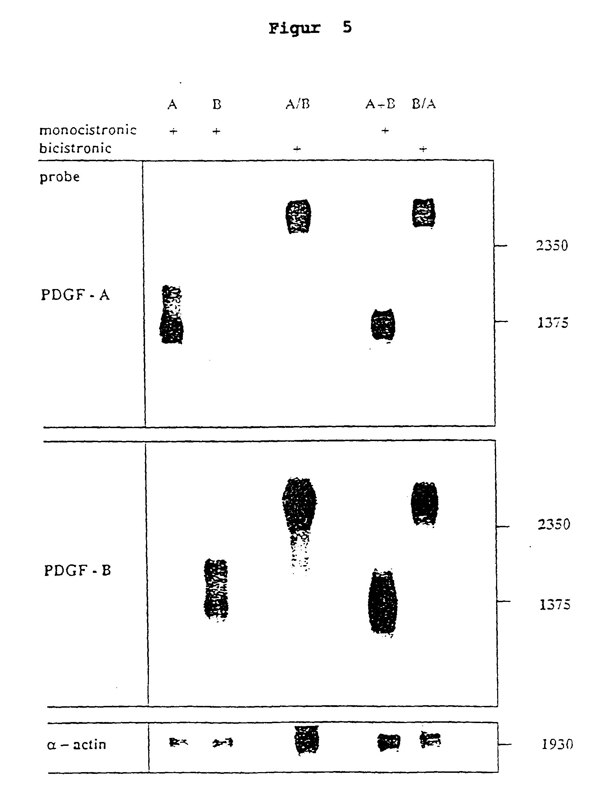

- Fig. 5): Northern-Blot Analyse transformierter BHK-Zellen. Es wurde die mRNA aus dem Gesamtpool der BHK-Zellen untersucht, die stabil mit den monocistronischen bzw. bicistronischen PDGF-Expressionskonstrukten transfiziert worden waren. Erwartungsgemäß zeigen die monocistronischen mRNAs eine Größe von etwa 1.300 nt, während bei den bicistronischen mRNAs die Größe der codierenden Sequenzen beider PDGF-Ketten (2.500 Nucleotide) vorhanden ist. Hiermit ist gezeigt, daß die entsprechenden Genprodukte von einer einzigen bicistronischen mRNA abgelesen werden. Als Referenz wurde die murine α-Actin Probe verwendet [Minty, A.J. et al., J. Biol. Chem. 256, 1008 - 1014, (1981)].
- Fig. 6): Sandwich-ELISA zum Nachweis von PDGF-AB mit Hilfe eines monoklonalen und eines polyklonalen Anti-PDGF-Antikörpers: Eichkurven von PDGF-Standards.
Polystyrolplatten wurden mit Schaf-Anti-Maus-IgG beschichtet und anschließend mit einem Maus-Hybridoma-Überstand (von Klon 1B3, enthält monoklonale Antikörper gegen die B-Kette in PDGF-AB und -BB) inkubiert; nach Inkubation mit verschiedenen PDGF Standards wurde das gebundene PDGF mit Hilfe eines polyklonalen Kaninchen-Anti-PDGF-AA, gefolgt von Peroxidase-markiertem Anti-Kaninchen-IgG nachgewiesen;
Quelle der PDGF-Standards:
AB: aus humanen Thrombozyten, von PROMEGA Corp. No. G 5161;
BB: rekombinant aus Hefe, von PROMEGA Corp. No. G 5191;
AA: rekombinant aus BHK-Zellen, ca. 70%^{ig} (Eichner et al., 1989).
Für PDGF's aus eukaryontischen Quellen ergibt sich ein spezifisches Signal mit PDGF-AB (aus humanen Thrombozyten) mit einer geringen Kreuzreaktion mit PDGF-BB.

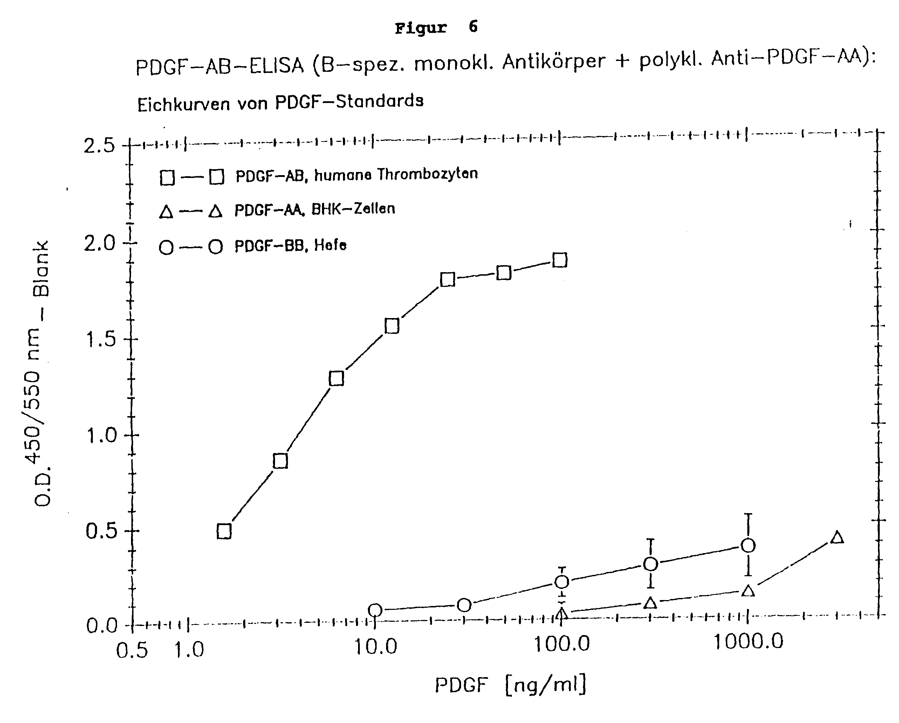

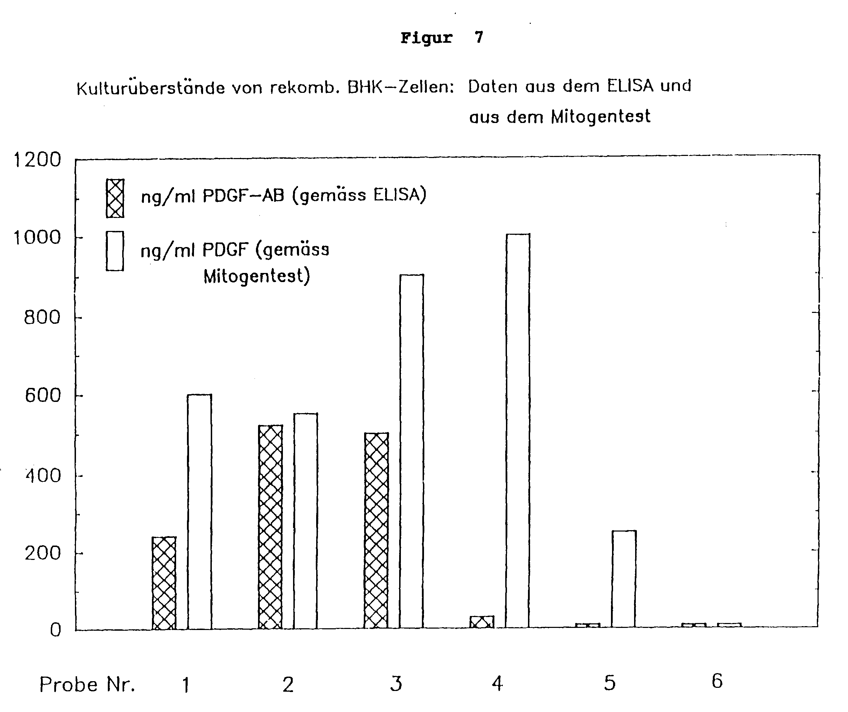

- Fig. 7): Nachweis von PDGF-AB in Kulturüberständen von rekombinanten BHK-Zellen mittels PDGF-AB-ELISA: (Eichkurven von Standards s. Fig. 6). Die dargestellten Proben stammten von BHK-Zellen, die mit folgenden Vektorkonstrukten transfiziert worden waren:
Probe: Nr.1: pSBC-A/B,
Nr.2: pSBC-B/A,
Nr.3: pSBC-2-PDGF-A +pSBC-2-PDGF-B,
Nr.4: pSBC-2-PDGF-A,
Nr.5: pSBC-2-PDGF-B,
Nr.6: pSBC-2-Kontrolle
- Fig. 8): Analyse von gereinigtem rPDGF über SDS-PAGE. Die Proben wurden auf einem 13,5%igen Polyacrylamidgel in der Gegenwart von SDS aufgetrennt und anschließend mit Coomassieblau gefärbt.
1, 6, 11 = LMW (PHARMACIA) [14400, 20100, 30000, 43000, 67000, 94000 D]
2 = pSBC-B/A
3 = pSBC-2-PDGF-A + pSBC-2-PDGF-B
4 = pSBC-A/B
5 = pSBC-2-PDGF-A
7-10 = gleiche Auftragsreihenfolge wie 2-5,unter Zugabe von jeweils 10% (v/v) β-Mercaptoethanol (10 min, 95°C)

Die Analyse der gereinigten Sekretionsprodukte über SDS-PAGE korreliert mit den Ergebnissen aus der Analyse der Kulturüberstände (Tab. 2). Insbesondere an den unter reduzierenden Bedingungen auftretenden Banden der PDGF-Monomere kann man gut erkennen, daß Transfektionszellpools der Konstellation pSBC-2PDGF-A + pSBC-2-PDGF-B (Cotransfer) und pSBC-A/B (PDGF-A-Kette im ersten Cistron) überwiegend PDGF-A&-Homodimere sekretieren.

Das aus pSBC-B/A-Kulturüberständen isolierbare PDGF bandiert gegenüber dem als Referenz aufgetragenen PDGF-AA bei einem geringfügig niedrigeren Molekulargewicht. Unter reduzierenden Bedingungen lassen sich beide Monomere zu etwa gleichen Teilen nachweisen. Die oberen Banden korrespondieren mit den Monomer-Banden der PDGF-A-Kette. Für rekombinantes PDGF-AA aus BHK-Zellen wurde bereits gezeigt, daß dieses Material zu etwa gleichen Anteilen aus der vollständigen PDGF-A-Kette, sowie einer C-terminal verkürzten Spezies besteht (Eichner et al., 1989).

Die monomeren PDGF-A-Ketten bandieren bei einer Mᵣ von etwa 17 KD. Darunter bandiert die PDGF-B-Kette (Mᵣ 16 KD), die bei dem aus pSBL-B/A-Überständen isolierten Material ebenfalls in einer verkürzten Form auftritt. Die Subspezies beider Ketten wurden gemeinsam durch Proteinsequenzanalyse analysiert und eindeutig als PDGF-A und PDGF-B identifiziert. Molekulargewichtsunterschiede für PDGF-B sind daher, genau wie bei PDGF-A, möglicherweise auf C-terminale Verkürzungen oder veränderte Glycosylierungsmuster zurückzuführen. PDGF aus Überständen von pSBC-B/A besteht demnach zu etwa gleichen Teilen aus PDGF-A und PDGF-B-Ketten.

Das als Referenz aufgetragene, ebenfalls in BHK-Zellen exprimierte PDGF-AA ist nicht glykosyliert (Eichner et al., 1989), während in CHO-Zellen exprimiertes PDGF einen Kohlenhydratanteil von etwa 7% enthält (Kolvenbach et al., 1991). Die PDGF-A-Monomere sowohl aus dem PDGF-AA-Homodimer als auch aus dem AB-Heterodimer stimmen in ihrem Laufverhalten in der SDS-PAGE gut überein.

### 2. Expression von PDGF-AB Heterodimer mit Hilfe des bicistronischen Vektorsystems

### 2.1 Herstellung der Basisvektoren pSBC-1 und pSBC-2 (Fig.1)

Für die Konstruktion des Vektors pSBC-1 wurde ein 627 bp MseI/BalI-Fragment aus dem Plasmid pGEM3-5' Polio (M) (Sarnow, 1989) als Matrize für eine PCR mit folgenden Primern verwendet: Das nach der Amplifikation erhaltene 652 bp Fragment wurde mit Pol I K behandelt, anschließend mit PstI gespalten und in den entsprechend präparierten Vektor pBEH (Artelt et al., 1988) insertiert.

Für die Konstruktion des Vektors pSBC-2 wurde das Plasmid pBEH mit Eco RI linearisiert und die folgenden Oligonukleotidsequenzen hybridisiert und insertiert:

### 2.2 Rekonstitution der vollständigen PDGF-B-Vorläufersequenz

Das Plasmid pMVW-2 enthält die cDNA des humanen PDGF-B Gens, welches im 5'-translatierten Bereich der Vorläufersequenz unvollständig ist (Weich et al., 1986). Für die Rekonstitution des authentischen PDGF-B precursors wurde in den 5'-terminalen Bereich des Vorläufers durch einen C-T-Austausch in Position 30 des codogenen Abschnittes des Klons pMVW-2 eine BclI-Schnittstelle eingeführt. Durch diesen Schritt geht letzlich nur ein kurzer Abschnitt des kodierenden Bereiches verloren und die lokal codierte Aminosäure (Asparaginsäure) bleibt dabei erhalten. Da die BclI-Schnittstelle in den meisten *E.coli-Stämmen* durch Methylierung resistent gegenüber enzymatischer Spaltung ist, muß das diese Schnittstelle enthaltene Fragment entweder in einen dam⁻-Stamm umkloniert, oder über einen PCR-Schritt amplifiziert werden. Der fehlende Bereich des Vorläufers wird dann als synthetisches SalI/BclI-Fragment [Oligomere PPDGFB1 und PPDGFB2 (SEQ ID Nr. 11 + 12)] eingesetzt.

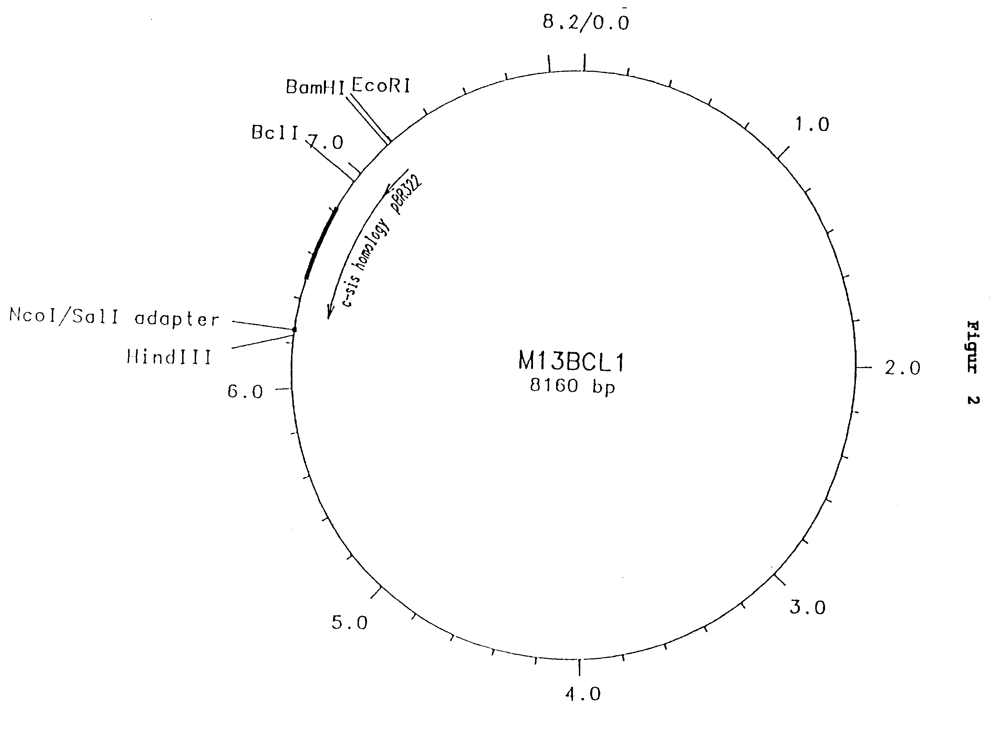

Hierfür wurde zunächst das 914 bp BamHI/NcoI-Fragment aus pMVW-2 über einen synthetischen Adapter [Oligomere NCCLSA1 und NCCLSA2 (SEQ ID Nr. 8 + 9)] in den BamHI/SalI-gespaltenen Bakteriophagen M13mp19 (Pharmacia) insertiert. Dieses Konstrukt lieferte die notwendige Einzelstrang-DNA für den nachgeschalteten *in vitro* Mutageneseschritt, der mit Hilfe des Oligomer-directed *in vitro* mutagenesis system (Version 2) der Fa. Amersham, basierend auf der Methode von Eckstein et al. [Taylor J. W., Ott J. and Eckstein F. (1985) Nucl. Acids Res. **13**, 8764-8785; Nakamaye K. and Eckstein F. (1986) Nucl. Acids Res. **14**, 9679-9698; Sayers J. R., Schmidt W. and Eckstein F. (1988) Nucl. Acids Res. **16**, 791-802] durchgeführt wurde. Durch den synthetischen Primer [PDGBBCL (SEQ ID Nr. 10)] wird nach der Mutagenese ein Basenaustausch (C zu T) in Position 144 der unter SEQ ID Nr. 3 dargestellten Sequenz erreicht und dadurch im 5'-Bereich des PDGF-B precursors eine BclI-Schnittstelle eingeführt. Dieses Mutagenesederivat wurde als M13BCL1 bezeichnet (Fig. 2).

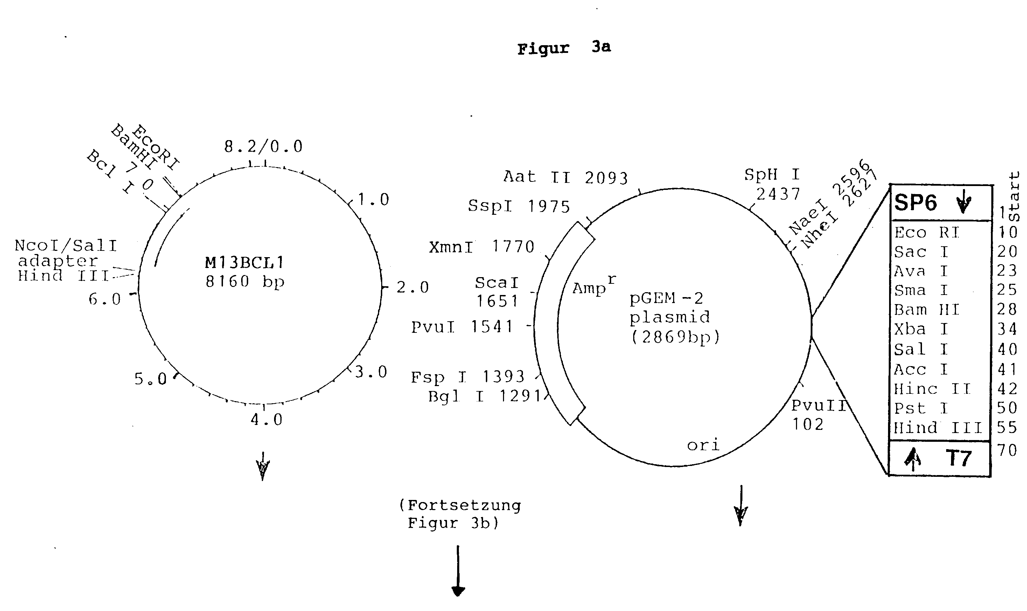

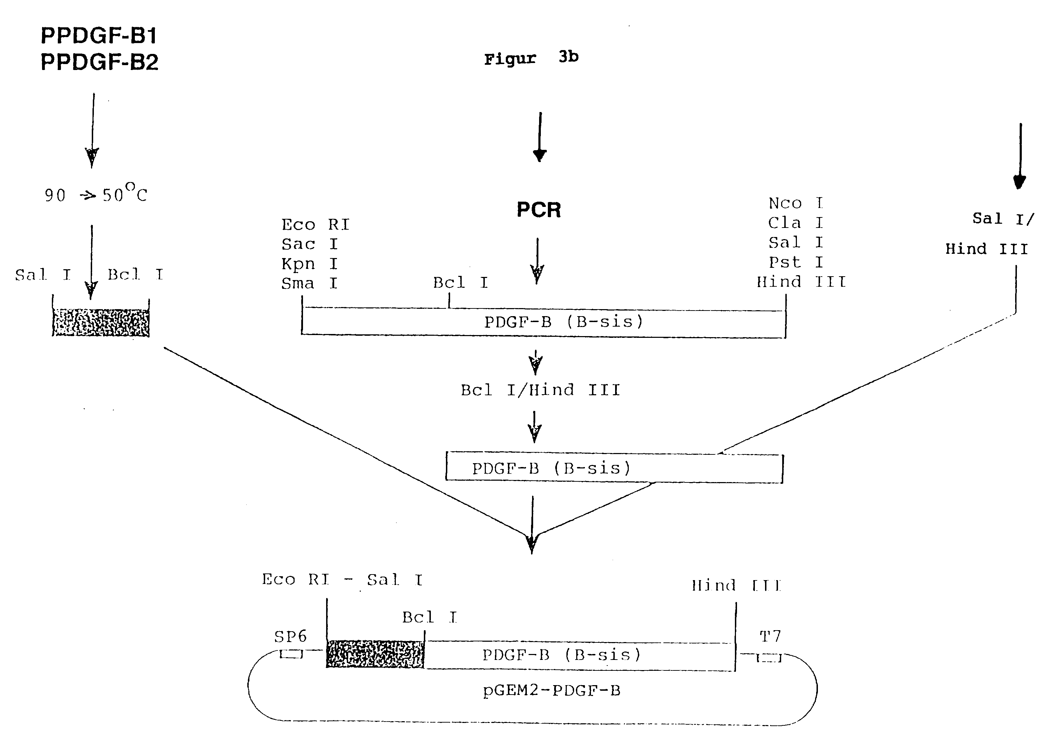

Ein 1100 bp Fragment aus M13BCL1 wurde über einen PCR-Schritt mit Hilfe der Primer M1317MER und M1324MER (SEQ ID Nr. 6 + 7) amplifiziert, anschließend einer BclI/HindIII-Restriktion unterworfen und das resultierende 770 bp Fragment isoliert. Die synthetischen Oligomere PPDGFB1 und PPDGFB2 (SEQ ID Nr. 11 + 12) bilden den fehlenden 5'-Bereich des PDGF-B-Vorläufers bis zur BclI-Schnittstelle. Nach dem Annealing wurde dieses doppelsträngige Oligomer anschließend, zusammen mit dem 770 bp PDGF-B Fragment, in den mit einer SalI/HindIII Restriktion vorbereiteten Vektor pGEM-2 (Promega) ligiert (Fig. 3). Die authentische Sequenz von PDGF-B wurde durch vollständige Sequenzierung verifiziert.

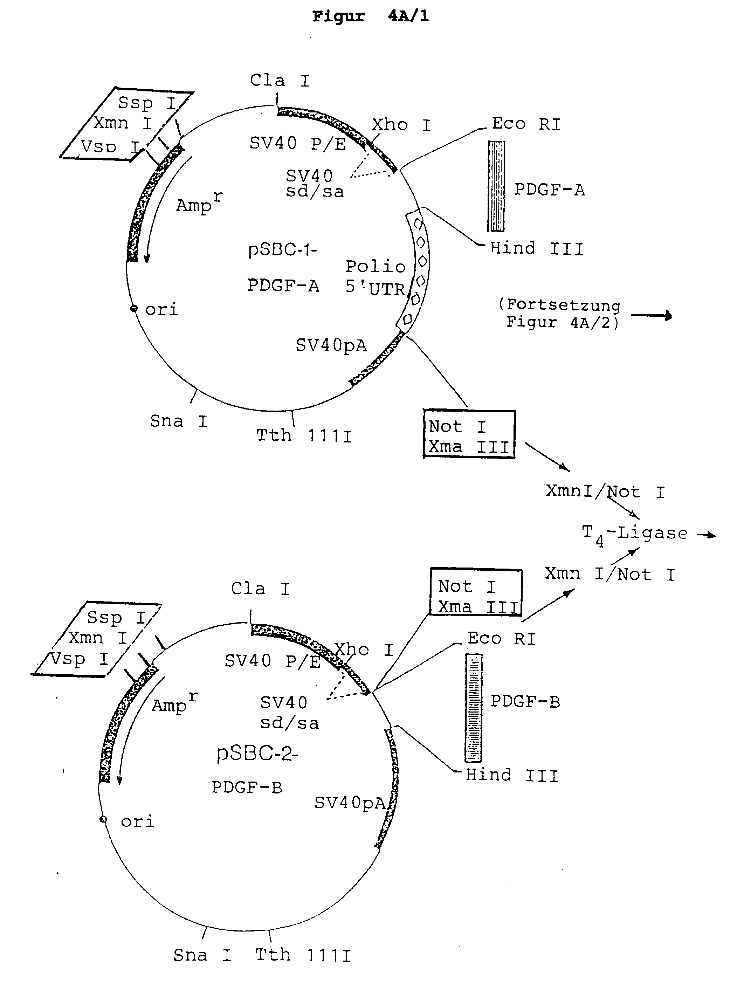

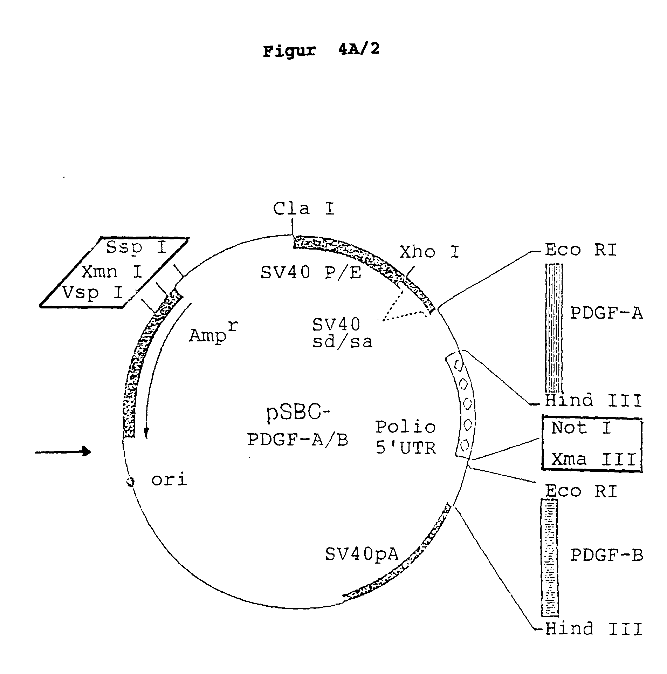

### 2.3 Konstruktion der bicistronischen Expressionsvektoren pSBC-PDGF-A/B und DSBC-PDGF-B/A für die PDGF-A- und B-Kette (Fig. 4A und B)

Die vollständige codierende cDNA für den PDGF-B precursor (Ratner et al., 1985) liegt in dem Vektor pGEM2-PDGF-B vor, wie unter 1.2 beschrieben. Die vollständige cDNA-Sequenz der kurzen Variante der PDGF-A-Kette (Betsholtz et al., 1986) ist im Expressionsvektor pODA (Eichner et al., 1989) enthalten. Dieser Vektor wurde erhalten durch Klonierung des RsaI-Fragments aus pPGF-1 (Hoppe et al., 1987) in den SV-40 Expressionsvektor pBEH (Artelt et al., 1988).

Die kodierenden cDNA-Sequenzen der PDGF-A- und -B-Kette wurden unter Verwendung von EcoRI/HindIII Restriktionen in die monocistronischen Vektoren pSBC-1 und -2 insertiert (Abb. 4). Die Fusion beider Vektoren zu einer bicistronischen Expressionseinheit wurde mit Hilfe der Restriktionsenzyme XmnI/NotI durchgeführt.

### 2.4 Herstellung transformierter BHK-Zellen

Die Transfektion der mono- und bicistronischen Expressionsvektoren, die die codierenden Sequenzen der A- und B-Kette von PDGF tragen (vgl. Fig.1, 4A+B) wurde mit der Calciumphosphat-Präzipitationstechnik in BHK-Zellen durchgeführt (Wigler et al., 1979; Graham & van der Eb, 1973). Einen Tag vor der Transfektion wurden 2-3 x 10⁵ BHK-Zellen/24 cm² in neue Kulturflaschen umgesetzt. Vier Stunden vor der Transfektion wurde ein Medienwechsel mit DME-Medium durchgeführt. 5 µg der o. g. Plasmid-DNA zusammen mit 0,5 µg der Selektionsplasmide pAG60 und pSVpac (Colbère-Garapin, 1981; Vara et al., 1986), welche für ein Neomycinresistenzgen bzw. für eine Puromycin-Resistenz kodieren, in 250 µl 250 mM CaCl₂ suspendiert. Die Lösung wurde langsam unter ständiger Verwirbelung durch steril eingeblasene Luft zu 250 µl 2 x HEPES-Puffer (280 mM NaCl; 50 mM HEPES; 1,5 mM NaH₂PO₄ pH 7,1) gegeben und und das erhaltene Präzipitat dem Nährmedium zugesetzt. Zwei Tage nach der Transfektion wurde durch Medienwechsel von DME- auf Doppelselektionsmedium (5 µg/ml Puromycin; 500 µg/ml G418, Wirth et al., 1988) die Selektion auf stabil transfizierte Zellen begonnen und eine Population von PDGF sekretierenden Zellklonen erhalten. Ein repräsentatives Klongemisch dieser Zellen wurde bei der DSM am 11. 8. 1992 unter der Nummer DSM ACC2045 hinterlegt.

### 2.5 Northern Blot Analyse

Polyadenylierte RNA aus transformierten BHK-Zellen wurde nach Purchio et al., (1979) isoliert, über ein 1%iges Agarose-Formaldehydgel fraktioniert (Lehrach et al., 1977), auf eine Nylonmembran geblottet und mit [³²P]-markierten PDGF-A- und -B-Ketten spezifischen Sonden hybridisiert (Fig. 5).

### 2.6 Gewinnung konditionierter Zellkulturüberstände

Die Transformation der BHK-Zellen erfolgte analog 2.4. Nach dem Auszählen der Kolonien wurden die Zellen abtrypsinisiert, in frischem Selektionsmedium aufgenommen und auf eine Zellzahl von 10⁵ Zellen/ml eingestellt. Je 10 ml dieser Zellsuspension wurden in eine Flasche mit 65 cm² Bodenfläche überführt und für weitere 48 h kultiviert. Danach wurde das Medium entfernt und durch 10 ml Produktionsmedium (DMEM, ohne Serum und Selektions-Antibiotoka) ersetzt. Nach 24 h wurde das Medium abgenommen und durch serumhaltiges Selektionsmedium ersetzt. Die geernteten Überstände wurden bis zur Analyse bei -20°C gelagert. Zum Zeitpunkt der Ernte betrug die Zellzahl/Flasche 0.8-1.2x10⁷.

### 2.7 Nachweis von PDGF in den Kulturüberständen mit Hilfe des Mitogentests

Die Messung der Stimulierung der DNA-Syntheserate von dichtearretierten Fibroblasten erlaubt eine Bestimmung der mitogenen Aktivität des PDGF. Eine Unterscheidung zwischen den Isoformen ist dabei nicht möglich.

Der Assay wurde gemäß Shipley et al. (1984) mit AKR-2B-Mausfibroblasten in 24-Well-Platten durchgeführt. Reines PDGF zeigt in dem Test eine halbmaximale Stimulierung bei einer Konzentration von etwa 5 ng/ml. Dieser Wert wurde benutzt, um Produktivitäten zu bestimmen. Die Ergebnisse aus dem Mitogentest sind in Fig. 7 den Werten aus dem PDGF-AB-ELISA gegenübergestellt.

### 2.8 Nachweis von PDGF-AB Heterodimer in den Kulturüberständen mit Hilfe eines spezifischen PDGF-AB ELISA's

Es wurde ein 'two-antibody sandwich assay' aufgebaut, der eine spezifische Quantifizierung von PDGF-AB neben PDGF-AA und -BB erlaubt.

Sandwich-Assay mit Hilfe eines monoklonalen und eines polyklonalen Anti-PDGF-Antikörpers:

Polystyrolplatten mit 96 Kavitäten (Fa. Dynatech, U-Platte, No. M124B) werden in folgender Reihenfolge beschichtet (zwischen jedem Schritt jeweils 4 x Waschen mit PBS mit 0,05% Tween 20):
1) Schaf-Anti-Maus-IgG (Fa. Boehringer Mannheim, Nr. 1097 105), 3 µg/ml.
2) 1% BSA (Fa. E. Merck, Nr. 12018) in PBS, pH 7,5, 100 µl für 1 h bei R.T.
3) Maus-Hybridoma-Überstand von Klon 1B3 [erhalten durch Fusion von SP2/0-Myelomzellen mit Milzzellen von Mäusen, die mit rekomb. PDGF-AB (aus *E. coli* gemäß Hoppe et al. (1990) immunisiert worden waren], 2 µg/ml IgG2a/ml. Der monoklonale Antikörper bindet spezifisch an der B-Kette von PDGF-Dimeren.
4) PDGF-haltige Lösungen, verdünnt in PBS mit 0,1 % BSA und 0,05 % Tween 20 (PBS+), 50 µl für 1 h bei R.T.
5) Polyklonales Kaninchen-Anti-PDGF-AA-IgG (Fa. Genzyme, No. ZP-214, bindet an der A-Kette von dimerem PDGF), 2 µg/ml in PBS+, 50 µl für 1 h bei R.T.
6) POD-markiertes Ziege-Anti-Kaninchen IgG (Fa. Pierce, No. 31460), 0,1 µg/ml in PBS+, 50 µl für 1 h bei R.T., Detektion mit dem Substrat Tetramethylbenzidin gemäß E.S. BOS et al. (J.Immunoassay 2 (1981), 187-204).

### 2.8.1 Ergebnisse:

In der Abbildung 7 sind die Resultate von drei unterschiedlichen Analysen für PDGF aus Kulturüberständen rekombinanter BHK-Zellen dargestellt.

Der Mitogentest liefert einen brauchbaren Wert für die Gesamtmenge des in den Kulturüberständen vorhandenen rPDGF, ohne zwischen den verschiedenen Isoformen (PDGF-AA, AB oder BB) differenzieren zu können.

Der spezifische Anteil an heterodimerem PDGF-AB kann mit ausreichend hoher Genauigkeit durch den PDGF-AB-spezifischen ELISA bestimmt werden. Aus der Differenz dieser Analyse zum Ergebnis des Mitogentests kann der prozentuale Anteil von PDGF-Homodimeren rechnerisch ermittelt werden (Tabelle 1).

Das Ergebnis des ELISA's zeigt, daß nur in Kulturüberständen von transfizierten Zellinien des Typs pSBC-PDGF-B/A die meßbare biologische Aktivität im Mitogentest mit hohen PDGF-AB-Werten korreliert.

### 2.9 Reinigung des sekretierten PDGF-AB

Für die Reinigung der Sekretionsprodukte aus 1,5 - 2,5 Litern konditionierter Kulturüberstände der unterschiedlichen Transfektions-Zellpools wurde ein für die Reinigung von rPDGF-AA aus Zellkulturüberständen entwickeltes Verfahren angewendet (Eichner et al., 1989). Das nach dem HPLC-Schritt hoch- oder teilgereinigte PDGF wurde auf einem Polyacrylamidgel nach Laemmli (1970) in der Gegenwart von SDS aufgetrennt und nach anschließender Coomassieblaufärbung analysiert (Fig. 8).

### 2.10 Aminoterminale Sequenzierung von PDGF-Polypeptiden

Durch Proteinsequenzanalyse sollten beide PDGF-Ketten eindeutig identifiziert werden um auszuschließen, daß hier verkürzte, prozessierte Formen nur einer PDGF-Kette vorliegen (s. Fig. 8).

Die automatische Sequenzanalyse wurde am Modell 477A (Applied Biosystems) mit dem Zyklus BLOTT1 durchgeführt. Die Analyse der Phenylthiohydantoin-Aminosäuren-Derivate erfolgte auf dem online gekoppelten 120A PTH-Analyser.

Die Disulfidbrücken der Probe werden mit Dithiothreitol reduziert und mit 4-Vinylpyridin alkyliert. Sie wird auf einem horizontalen SDS-Elektrophorese-Gel nach Schägger und von Jagow, modifiziert wie beschrieben (Westermeier et al. SD 092/89, Pharmacia LKB Biotechnologie) getrennt. Die Probe wird auf eine PVDF-Membran (Problot, Applied Biosystems) mit einem diskontinuierlichen Puffersystem wie beschrieben (Westermeier et al. SDRE-072, Pharmacia LKB Biotechnologie) geblottet und mit Comassie Brillant Blau R250 gefärbt. Die beiden Doppelbanden bei 17 und 16 KD werden ausgeschnitten und zusammen sequenziert.

Nach den Ergebnissen der Proteinbestimmung wurden 10 µg Probe analysiert. Es konnten die N-terminalen Aminosäuren der PDGF-A- und PDGF-B-Kette in gleicher Ausbeute nachgewiesen werden (Tabelle 2). Nebensequenzen wurden nicht detektiert.

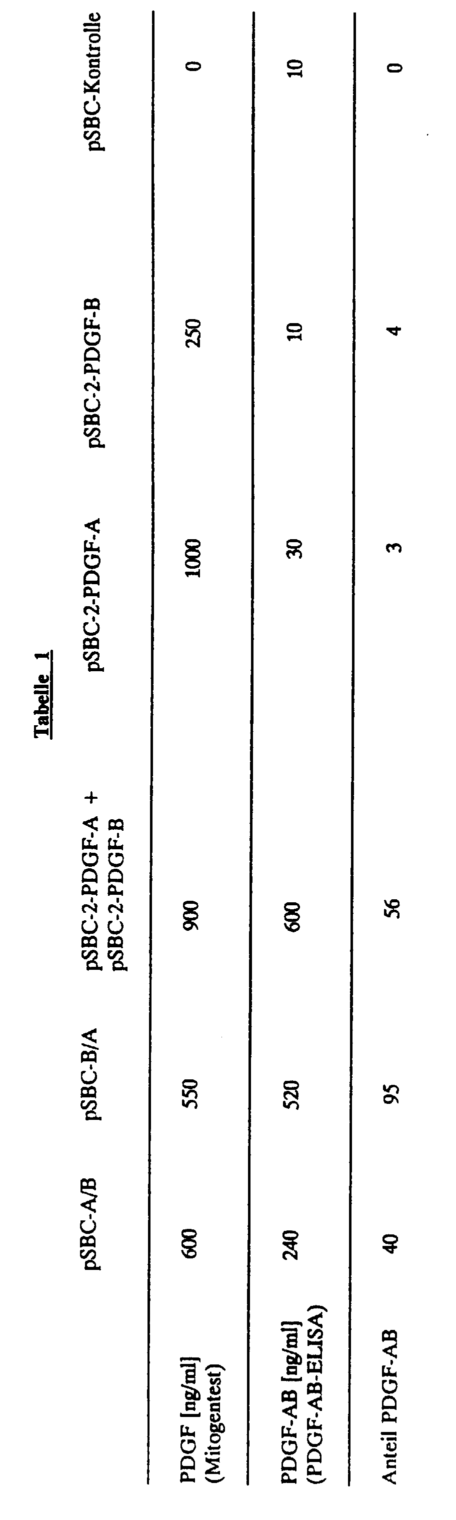

**Tabelle 2**

| Aminosäurensequenzanalyse der PDGF-A- und PDGF-B-Kette | | | | |
|---|---|---|---|---|
| Zyklus | PDGF-A | | PDGF-B | |
| | Code | Ausbeute (pmol) | Code | Ausbeute (pmol) |
| 1 | Ser | 101,1* | Ser | |
| 2 | Ile | 75,7 | Leu | 89,7 |
| 3 | Glu | 58,8 | Gly | 82,0 |
| 4 | Glu | 67,2 | Ser | 42,9 |
| 5 | Ala | 55,7 | Leu | 70,2 |
| 6 | Val | 61,0 | Thr | 59,4 |
| 7 | Pro | 45,9 | Ile | 65,4 |
| 8 | Ala | 104,6* | Ala | |
| 9 | Val | 46,8 | Glu | 49,9 |
| 10 | Cys | 40,5 | Pro | 31,8 |
| 11 | Lys | 24,1 | Ala | 34,6 |
| 12 | Thr | 23,5 | Met | 16,5 |
| 13 | Arg | 30,3 | Ile | 25,2 |
| 14 | Thr | 24,7 | Ala | 29,2 |
| 15 | Val | 17,5 | Glu | 28,6 |
| 16 | Ile | 27,5 | Cys | 23,2 |
| 17 | Tyr | 16,4 | Lys | 11,2 |
| 18 | Glu | 20,9 | Thr | 13,4 |
| 19 | Ile | 24,8 | Arg | 20,9 |
| 20 | Pro | 17,1 | Thr | 16,9 |
| 21 | Arg | 29,0 | Glu | 16,7 |
| 22 | | | Val | 19,8 |
| 23 | Gln | 8,1 | Phe | 10,2 |

| | | | | |
|---|---|---|---|---|
| * Ausbeute aus beiden Ketten | | | | |

### Abkürzungen:

- BHK -: Hamsterzellinie (Baby Hamster Kidney)
- bp -: Basenpaar(e)
- CHO -: Hamsterzellinie (Chinese Hamster Ovary)
- BSA -: Rinderserumalbumin
- D -: Dalton
- DMEM -: Dulbecco's Modified Eagle Medium
- ELISA -: enzyme-linked immunosorbent assay
- HEPES -: 4-(2-Hydroxyl)-1-piperazinethansulfonsäure
- HPLC -: Hochdruckflüssigkeitschromatographie
- IgG -: Immunglobulin der Klasse G
- IRES -: internal ribosomal entry site
- nt -: Nukleotid(e)
- PAGE -: Polyacrylamid-Gelelektrophorese
- PBS -: phosphatgepufferte Kochsalzlösunq
- PCR -: Polymerase Kettenreaktion
- PDGF -: Wachstumsfaktor aus Thrombozyten
- POD -: Peroxidase
- PVDF -: Polyvinylidenfluorid
- SDS -: Natriumdodecylsulfat
- UTR -: nicht translatierte Region

### LITERATUR

**Adam M. A., Ramesh N., Miller A. D., and Osborne W. R. A.** (1991) J. Virol. **65**, 4985-4990.
**Artelt P., Morelle C., Ausmeier M., Fitzek M., and Hauser H.** (1988) Gene **68**, 213-219.
**Beckmann M. P., Betsholtz C., Heldin C.-H., Westermark B., Di Marco E., Di. Fiore P. P., Robbins K. C., and Aaronson S. A.** (1988) Science **241**, 1344-1349.
**Betsholtz C., Johnsson A., Heldin C.-H., Westermark B., Lind P., Urdea M. S., Eddy R., Shows T. B., Philpott K., Mellor A. L., Knott T. J., and Scott J.** (1986) Nature **320**, 695-699.
**Block L. H., Emmons L. R., Vogt E., Sachinidis A., Vetter W., and Hoppe J.** (1989) Proc. Natl. Acad. Sci. USA **86**, 2388-2392.
**Boel E., Berkner K. L., Nexoe B. A., and Schwartz T. W.** (1987) FEBS Lett. **219**, 181-188.
**Bywater M., Rorsman F., Bongcam-Rudloff E., Mark G., Hammacher A., Heldin C.-H., Westermark B., and Betsholtz C.** (1988) Mol. Cell. Biol. **8**, 2753-2762.
**Colbére-Garapin F., Horodniceanu F., Kourilsky P., and Garapin A. C.** (1981) J. Mol. Biol. **150**, 1-14.
**Eichner W., Jäger V., Herbst D., Hauser H. and Hoppe J.** (1989) Eur. J. Biochem. **185**, 135-140.
**Falcone D., and Andrews D.W.** (1991) Mol. Cell. Biol. **11** (5), 2656-2664.
**Ghattas I. R., Sanes J. R., and Majors J. E.** (1991) Mol. Cell. Biol. **22**, 5848-5859.
**Graham F., and van der Eb L.** (1973) Virology **52**, 456-487.
**Hambidge S. J., and Sarnow P.** (1991) J. Virol. **65**, 6312-6315.
**Hammacher A., Hellmann U., Johnsson A., Östman A., Gunnarsson K., Westermark B.**, **Wasteson Å., and Heldin C.-H.** (1988) J. Biol. Chem. **263**, 16493-16499.
**Hart C. E., Forstrom J. W., Kelly J. D., Seifert R. A., Smith R. A., Ross R., Murray M. J., and Bowen-Pope D. F.** (1988) Science **240**, 1529-1531.
**Hart C. E., Bailey M., Curtis D. A., Osborn S., Raines E., Ross R., and Forstrom J. W.** (1990) Biochemistry **29**, 166-172.
**Heldin C.-H., Johnsson A., Wennergren S., Wernstedt C., Betsholtz C., and Westermark B.** (1986) Nature **319**, 511-514.
**Heldin C.-H., Bäckström G., Östman A., Hammacher A., Rönnstrand L., Rubin K., Nister M., and Westermark B**. (1988) EMBO J. **7**, 1387-1393.
**Hoppe J., Schumacher** L., **Eichner W. and Weich H.A.** (1987), FEBS Lett. **223**, 243-246.
**Hoppe J., Weich H. A., and Eichner W.** (1989) Biochemistry **28**, 2956-2960.
**Hoppe J., Weich H. A., and Eichner W., and Tatje D.** (1990) Eur. J. Biochem. **187**, 207-214.
**Hosang M., Rouge M., Wipf B., Eggiman B., Kaufmann F., and Hunziker W.** (1989) J. Cell. Physiol. **149**, 558-564.
**Jackson R. J., Howell M.** T., **and Kaminski** A. (1990) Trends Biochem. Sci. **15**, 477-483.
**Jang S. K., Kräusslich H., Nicklin M. J. H., Duke G. M., Palmenberg A. C., and Wimmer E.** (1988) J. Virol. **62**, 2636.
**Jang S. K., Davies M. V., Kaufmam R. J., and Wimmer E.** (1989) J. Virol. **63** (4), 1651-1660.
**Jang S. K.,** and **Wimmer E.** (1990) Genes Dev. **4**, 1560-1572.
**Johnsson A., Heldin C.-H., Wasteson A., Westermark B., Deuel T. F., Huang J. S., Seeburg P. H., Gray A., Ullrich A., Scrace G., Stroobant P., Waterfield M. D.** (1984) EMBO J. **136**, 921-928.
**Kaufman R. J., Murtha P., and Davies M. V.** (1987) EMBO J. **6**, 187-193.
**Kaufman R. J., Davies M. V. Wasley L. C., and Michnick D.** (1991) Nucleic Acids Res. **19**, 4485-4490.
**Kelly J. D., Raines E. W., Ross R., and Murray M. J.** (1985) EMBO J. **4**, 3399-3405.
**Kolvenbach C. G., Langley K. E., Strickland T. W., Kenney W. C., and Arakawa** T. (1991) J. Biochem. Biophys. Meth. **23**, 295-300.
**Kozak M.** (1987) Mol. Cell. Biol. **7** (10), 3438-3445.
**Kozak M**. (1989) Mol. Cell. Biol. **9**, 5134-5142.
**Laemmli U. K.** (1970) Nature **227**, 680-685.
**Lehrach H., Diamond D., Wozney J. M., and Boedtker H.** (1977) Biochemistry **16**, 4743-4751.
**Macejak D. G., and Sarnow P.** (1991) Nature (London) **353**, 90-94.
**Matoskova B., Rorsman F., Svensson V. and Betsholtz C.** (1989), Mol. Cell. Biol. **9**, 3148-3150.
**Meerovitch K., Pelletier J., and Sonenberg N.** (1989) Genes Dev. **3**, 1026-1034.
**Nister M., Hammacher A., Mellström K., Siegbahn A., Rönnstrang L., Westermark B., and Heldin C.-H.** (1988); Cell **52**, 791-799.
**Östman A., Rall L., Hammacher A., Wormstead M. A., Coit D., Valenzuela P., Betsholtz C., Westermark B., and Heldin C.-H.** (1988) J. Biol. Chem. **263**, 16202-16208.
**Pelletier J., and Sonenberg N.** (1988) Nature **334**, 320.
**Purchio A. F. and Fareed G. C.** (1979) J. Virol. **29,** 763-769.
**Ratner L., Josephs S. F., Jarrett R., Reitz M. S. and Wong-Staal** F. (1985), Nucl. Acids Res. **13**, 5007-5018.
**Reilly C. F. and Broski J. E.** (1989) Biochem. Biophys. Res. Commun. **160**, 1047-1054.
**Sachinidis A., Locher R., Vetter W., Tatje D., and Hoppe J.** (1990) J. Biol. Chem. **265**, 10238-10243.
**Sachinidis A., Locher R., Hoppe J., and Vetter W.** (1990) FEBS Lett. **275**, 95-98.
**Sarnow P.** (1989) J. Virol. **63**, 467-470.
**Shipley G. D., Childes C. B., Volkenant M. E. and Moses H. L.** (1984) Cancer Res. **44**, 710-716.
**Siegbahn A., Hammacher A., Westermark B., and Heldin C.-H.** (1990) J. Clin. Invest. **85**, 916-920.
**Simoes E. A. F., and Sarnow P.** (1991) J. Virol. **65**, 913-921.
**Stroobant P., and Waterfield M. D.** (1984) EMBO J. **3**, 2963-2967.
**Vara J., Portela A., Oritin J. and Jimenez A.** (1986) Nucl. Acids Res. **14**, 4617-4624.
**Weich H. A., Sebald W., Schairer H. U., and Hoppe J.** (1986), FEBS Lett. **198**, 344-348.
**Wigler M., Sweet R., Sim G. K., Wold B., Pellicer A., Lacy E., Maniatis T., Silverstein S., and Axel R.** (1979) Cell **16**, 777-785.
**Wirth M., Bode J., Zettlmeißl G., and Hauser H.** (1988) Gene **73**, 419-426.
**Wirth M., Schumacher L., and Hauser H.** (1991) In Modern Approaches to Animal Cell Technology, Griffiths B., Spier R., and Meigner R., eds. Butterworths), pp. 338-343.
**Wise R. J., Orkin S. H. and Collins T.** (1989) Nucl. Acids Res. **17**, 6591-6601.
**Wood C. R., Morris G. E., Alderman E. M., Fouser L., and Kaufman R. J.** (1991) Proc. Natl. Acad. Sci. USA **88**, 8006-8010.
**Young R. M., Mendoza A. E., Collins T. and Orkin S. H.** (1990) Mol. Cell. Biol. **10**, 6051-6054.

## Patentansprüche

1. Bicistronische Expressionseinheit zur rekombinanten Herstellung von heterodimerem PDGF-AB (Platelet Derived Growth Factor AB) in Säugerzellen als Wirtszellen, gekennzeichnet durch die allgemeine Formel
p - 5'UTR - C₁ - IRES - C₂ - 3'UTR - polyA,
in der
p ein transkriptionaler Promotor ist,
5'UTR (5'untranslated region) eine nicht translatierte Nukleotidsequenz ist,
C₁ ein Cistron ist, welches ein für die B-Kette von PDGF, ein biologisch aktives Analogon oder ein biologisch aktives Fragment derselben kodierendes Gen enthält,
IRES (Internal Ribosomal Entry Site) eine Nukleotidsequenz viralen, zellulären oder synthetischen Ursprungs ist, die eine interne Bindung der Ribosomen vermittelt;
C₂ ein Cistron ist, welches ein für die A-Kette von PDGF oder ein biologisch aktives Analogon oder ein biologisch aktives Fragment derselben kodierendes Gen enthält,
3'UTR (3'untranslated region) eine nicht translatierte Nukleotidsequenz ist,
und polyA ein Polyadenylierungssignal ist,
wobei C₁, IRES und C₂ operativ miteinander verknüpft sind.

2. Expressionseinheit nach Anspruch 1, **dadurch gekennzeichnet**, daß C₁ die vollständige PDGF-B Vorläufersequenz (SEQ ID Nr. 3), deren allelische Varianten oder Fragmente derselben enthält, welche für eine biologisch wirksame PDFG-B Kette kodiert.

3. Expressionseinheit nach Anspruch 2, **dadurch gekennzeichnet**, daß C₁ das *v-sis-Gen* aus Simian Sarcoma Virus oder die Basenpaare 283 bis 609 gemäß SEQ ID Nr. 3 enthält.

4. Expressionseinheit nach den Ansprüchen 1 bis 3, **dadurch gekennzeichnet**, daß C₂ die PDGF-A_{K}- (SEQ ID Nr. 1) oder die PDGF-A_{L} Vorläufer-Sequenz enthält.

5. Expressionseinheit nach den Ansprüchen 1 bis 4, **dadurch gekennzeichnet**, daß IRES die Nukleotidsequenz gemäß SEQ ID Nr. 5 ist.

6. Expressionseinheit nach den Ansprüchen 1 bis 4, **dadurch gekennzeichnet**, daß IRES die 5'UTR des Enzephalomyocarditis Virus (EMV), des "Theilers murine encephalomyelitis virus" (TMEV), des "foot and mouth disease virus" (FMDV), des "bovine enterovirus" (BEV), des "coxsackie B virus" (CBV), des "human rhinovirus" (HRV) oder die "human immunoglobulin heavy chain binding protein" (BIP) 5'UTR, die Drosophila Antennapediae 5'UTR, die Drosophila Ultrabithorax 5'UTR oder genetische Hybride oder Fragmente aus den oben angeführten Sequenzen ist, wobei die genetischen Hybride oder Fragmente eine interne Bindung der Ribosomen vermitteln.

7. Rekombinanter DNA-Vektor, welcher die Expressionseinheit nach den Ansprüchen 1 bis 6, operativ verknüpft mit Expressionskontrollsequenzen, enthält.

8. Wirtszelle, welche eine Säugerzelle transformiert mit dem Vektor gemäß Anspruch 7 ist.

9. Wirtszelle nach Anspruch 8, **dadurch gekennzeichnet**, daß sie eine CHO- oder BHK-Zelle ist.

10. Wirtszelle nach Anspruch 9, **dadurch gekennzeichnet**, daß sie eine BHK-Zelle ist und von dem Klon 91-21-4D mit der Hinterlegungs-Nr. DSM ACC2045 abstammt.

11. Verfahren zur Herstellung von heterodimerem rPDGF-AB, **dadurch gekennzeichnet**, daß man Säugerzellen als Wirtszellen, die eine Expressionseinheit nach den Ansprüchen 1 bis 6 operativ insertiert enthalten, in einem geeigneten Medium kultiviert und das so erzeugte rPDGF-AB von den Zellen und dem Medium abtrennt.

12. Verfahren nach Anspruch 11, **dadurch gekennzeichnet**, daß die Wirtszelle eine Zelle nach den Ansprüchen 9 bis 11 ist.

13. Verfahren zur Herstellung eines rPDGF-AB enthaltenden pharmazeutischen und/oder kosmetischen Präparates, dadurch gekennzeichnet, daß man das Verfahren nach den Ansprüchen 11 oder 12 durchführt und das abgetrennt rPDGF-AB in einem weiteren Schritt zusammen mit pharmazeutisch und/oder kosmetisch verträglichen Hilfs- und Trägerstoffen formuliert.

14. Verfahren nach Anspruch 13, dadurch gekennzeichnet, daß das pharmazeutische und/oder kosmetische Präparat eine Salbe, ein Gel, ein Spray, ein Pflaster, ein Wundverband oder eine Wundauflage ist.

## Claims

1. Bicistronic expression unit for the recombinant preparation of heterodimeric PDGF-AB (platelet derived growth factor AB) in mammalian cells as host cells, characterized by the general formula
p - 5'UTR - C₁ - IRES - C₂ - 3'UTR - polyA
in which
p is a transcriptional promoter,
5'UTR (5' untranslated region) is a non-translated nucleotide sequence,
C₁ is a cistron which contains a gene coding for the B chain of PDGF, a biologically active analogue or a biologically active fragment thereof,
IRES (internal ribosomal entry site) is a nucleotide - sequence of viral, cellular or synthetic origin, which mediates internal binding of the ribosomes,
C₂ is a cistron which contains a gene coding for the A chain of PDGF or a biologically active analogue or a biologically active fragment thereof,
3'UTR (3' untranslated region) is a non-translated nucleotide sequence,
and polyA is a polyadenylation signal,
where C₁, IRES and C₂ are linked together in an operative manner.

2. Expression unit according to Claim 1, characterized in that C₁ contains the complete PDGF-B precursor sequence (SEQ ID No. 3), its allelic variants or fragments thereof, which codes for a biologically active PDFG-B chain.

3. Expression unit according to Claim 2, characterized in that C₁ contains the *v-sis* gene from simian sarcoma virus or base pairs 283 to 609 according to SEQ ID No. 3.

4. Expression unit according to Claims 1 to 3, characterized in that C₂ contains the PDGF-A_{K} (SEQ ID No. 1) or the PDGF-A_{L} precursor sequence.

5. Expression unit according to Claims 1 to 4, characterized in that IRES is the nucleotide sequence according to SEQ ID No. 5.

6. Expression unit according to Claims 1 to 4, characterized in that IRES is the 5'UTR of encephalomyocarditis virus (EMV), of Theilers murine encephalomyelitis virus (TMEV), of foot and mouth disease virus (FMDV), of bovine enterovirus (BEV), of coxsackie B virus (CBV), of human rhinovirus (HRV) or the human immunoglobulin heavy chain binding protein (BIP) 5'UTR, the Drosophila Antennapediae 5'UTR, the Drosophila Ultrabithorax 5'UTR or genetic hybrids or fragments from the abovementioned sequences, where the genetic hybrids or fragments mediate internal binding of the ribosomes.

7. Recombinant DNA vector which contains the expression unit according to Claims 1 to 6, linked in an operative manner to expression control sequences.

8. Host cell which is a mammalian cell transformed with the vector according to Claim 7.

9. Host cell according to Claim 8, characterized in that it is a CHO or BHK cell.

10. Host cell according to Claim 9, characterized in that it is a BHK cell and is derived from the clone 91-21-4D with the deposit No. DSM ACC2045.

11. Process for preparing heterodimeric rPDGF-AB, characterized in that mammalian cells, as host cells, which contain an expression unit according to Claims 1 to 6 inserted in an operative manner are cultivated in a suitable medium, and the rPDGF-AB produced in this way is separated off from the cells and the medium.

12. Process according to Claim 11, characterized in that the host cell is a cell according to Claims 9 to 11.

13. Process for preparing an rPDGF-AB-containing pharmaceutical and/or cosmetic preparation, characterized in that the process according to Claims 11 or 12 is carried out and the rPDGF-AB which has been separated off is formulated in a further step together with pharmaceutically and/or cosmetically suitable auxiliaries and excipients.

14. Process according to Claim 13, characterized in that the pharmaceutical and/or cosmetic preparation is an ointment, a gel, a spray, a plaster, a wound bandage or a wound dressing.

## Revendications

1. Unité d'expression bicistronique pour la production recombinante de PDGF-1B (facteur de croissance dérivé des plaquettes AB) hétérodimère dans des cellules de mammifère en tant que cellules hôtes, caractérisée par la formule générale
P-5'UTR-C₁-IRES-C₂-3'UTR-polyA,
dans laquelle
p est un promoteur transcriptionnel,
5'UTR (région non traduite en 5') est une séquence nucléotidique non traduite,
C₁ est un cistron qui contient un gène codant pour la chaîne B de PDGF, un analogue biologiquement actif ou un fragment biologiquement actif de celle-ci,
IRES (site d'entrée ribosomique interne) est une séquence nucléotidique d'origine virale, cellulaire ou synthétique, qui médie une fixation interne des ribosomes,
C₂ est un cistron qui contient un gène codant pour la chaîne A de PDGF ou un analogue biologiquement actif ou un fragment biologiquement de celle-ci,
3'UTR (région non traduite en 3') est une séquence nucléotidique non traduite,
et polyA est un signal de polyadénylation,
C₁, IRES et C₂ étant fonctionnellement liés les uns aux autres.

2. Unité d'expression selon la revendication 1, caractérisée en ce que C₁ contient la séquence de précurseur complète de PDGF-B (SEQ ID n° 3), ses variantes allèles ou des fragments de celle-ci, codant pour une chaîne B de PDGF biologiquement active.

3. Unité d'expression selon la revendication 2, caractérisée en ce que C₁ contient le gène *v-cis* provenant du virus de sarcome simien ou les paires de bases 283 à 609 selon SEQ ID n° 3.

4. Unité d'expression selon les revendications 1 à 3, caractérisée en ce que C₂ contient la séquence de précurseur PDGF-A_{K} (SEQ ID n° 1) ou la séquence de précurseur PDGF-A_{L}.

5. Unité d'expression selon les revendications 1 à 4, caractérisée en ce que IRES est la séquence nucléotidique selon SEQ ID n° 5.

6. Unité d'expression selon les revendications 1 à 4, caractérisée en ce que IRES est la séquence 5'UTR du virus de l'encéphalomyocardite (EMV) du virus de l'encéphalomyélite murine de Theiler (TMEV), du virus de la fièvre aphteuse (FMDV), de l'entérovirus bovin (BEV), du virus coxsackie B (CBV), du rhinovirus humain (HRV) ou la séquence 5'UTR de protéine de liaison à la chaîne lourde d'immunoglobuline humaine (BIP), la séquence 5'UTR d'Antennapediae de drosophile, la séquence 5'UTR *d'Ultrabithorax* de drosophile ou des fragments ou hybrides génétiques des séquences indiquées ci-dessus, les fragments ou hybrides génétiques médiant une fixation interne des ribosomes.

7. Vecteur d'ADN recombinant qui contient l'unité d'expression selon les revendications 1 à 6, fonctionnellement lié à des séquences régulatrices d'expression.

8. Cellule hôte, qui est une cellule de mammifère transformée par le vecteur selon la revendication 7.

9. Cellule hôte selon la revendication 8, caractérisée en ce qu'elle est une cellule CHO ou BHK.

10. Cellule hôte selon la revendication 9, caractérisée en ce qu'elle est une cellule BHK et dérive du clone 91-21-4D portant le numéro de dépôt DSM ACC2045.

11. Procédé pour la production de rPDGF-AB hétérodimère, caractérisé en ce que l'on cultive dans un milieu approprié des cellules de mammifèreen tant que cellules hôtes, qui contiennent, fonctionnellement insérée, une unité d'expression selon les revendications 1 à 6, et le rPDGF-AB ainsi produit est séparé des cellules et du milieu.

12. Procédé selon la revendication 11, caractérisé en ce que la cellule hôte est une cellule selon les revendications 9 à 11.

13. Procédé pour la fabrication d'une préparation pharmaceutique et/ou cosmétique contenant rPDGF-AB, caractérisé en ce que l'on met en oeuvre le procédé selon la revendication 11 ou 12 et on formule le rPDGF-AB séparé, dans une autre étape, conjointement avec des adjuvants et véhicules pharmaceutiquement et/ou cosmétiquement acceptables.

14. Procédé selon la revendication 13, caractérisé en ce que la préparation pharmaceutique et/ou cosmétique est une pommade, un gel, une composition pour aérosol, un emplâtre, un bandage ou un pansement.


### SEQUENZPROTOKOLL

(1) ALLGEMEINE INFORMATION:
   (i) ANMELDER:
      (A) NAME: Beiersdorf-AG
      (B) STRASSE: Unnastr. 48
      (C) ORT: Hamburg
      (E) LAND: Bundesrepublik Deutschland
      (F) POSTLEITZAHL: 20245

      (A) NAME: GBF - Gesellschaft fuer Biotechnologische
         Forschung mbH
      (B) STRASSE: Mascheroder Weg 1
      (C) ORT: Braunschweig
      (E) LAND: Bundesrepublik Deutschland
      (F) POSTLEITZAHL: 38124
   (ii) ANMELDETITEL: Herstellung von heterodimerem PDGF-AB mit Hilfe eines bicistronischen Vektorsystems in Saeugerzellen
   (iii) ANZAHL DER SEQUENZEN: 16
   (iv) COMPUTER-LESBARE FORM:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.25 (EPA)
(2) INFORMATION ZU SEQ ID NO: 1:
   (i) SEQUENZ CHARAKTERISTIKA:
      (A) LÄNGE: 748 Basenpaare
      (B) ART: Nukleinsäure
      (C) STRANGFORM: Einzel
      (D) TOPOLOGIE: linear
   (ii) ART DES MOLEKÜLS: cDNS
   (vi) URSPRÜNGLICHE HERKUNFT:
      (A) ORGANISMUS: Homo sapiens
   (vii) UNMITTELBARE HERKUNFT:
      (B) CLON: pODA (Eichner et al., 1989)
   (ix) MERKMALE:
      (A) NAME/SCHLÜSSEL: CDS
      (B) LAGE: 95..682
      (D) SONSTIGE ANGABEN: /product= "PDGF-A Vorlaeufersequenz
         (kurze Spliceform)"
         /note= "humanes PDGF-A Gen (kurze Spliceform, [2])
         aus pODA, flankiert von 5'-EcoRI und 3'-HindIII
         Restriktionsschnittstellen"
         /citation= ([2])
   (ix) MERKMALE:
      (A) NAME/SCHLÜSSEL: mat_peptide
      (B) LAGE: 353..682
      (D) SONSTIGE ANGABEN: /product= "mature PDGF-A Kette"
   (x) VERÖFFENTLICHUNGSINFORMATION:
      (A) AUTOREN: Eichner, W.
         Jaeger, V.
         Herbst, D.
         Hauser, H.
         Hoppe, J.
      (C) ZEITSCHRIFT: Eur. J. Biochem.
      (D) BAND: 185
      (F) SEITEN: 135-140
      (G) DATUM: 1989
   (x) VERÖFFENTLICHUNGSINFORMATION:
      (A) AUTOREN: Hoppe, J.
         Schumacher, L.
         Eichner, W.
         Weich, H. A.
      (C) ZEITSCHRIFT: FEBS Lett.
      (D) BAND: 223
      (F) SEITEN: 243-246
      (G) DATUM: 1987
   (xi) SEQUENZBESCHREIBUNG: SEQ ID NO: 1:
   GAATTCCCAC TGAATTTCGC CGCCACAGGA GACCGGCTGG AGCGCCCGCC CCGCGCCTCG      60
   CCTCTCCTCC GAGCAGCCAG CGCCTCGGGA CGCG ATG AGG ACC TTG GCT TGC         112
                                         Met Arg Thr Leu Ala Cys
                                         -86 -85
   CTG CTG CTC CTC GGC TGC GGA TAC CTC GCC CAT GTT CTG GCC GAG GAA       160
   Leu Leu Leu Leu Gly Cys Gly Tyr Leu Ala His Val Leu Ala Glu Glu
   -80                 -75                 -70                 -65
   GCC GAG ATC CCC CGC GAG GTG ATC GAG AGG CTG GCC CGC AGT CAG ATC       208
   Ala Glu Ile Pro Arg Glu Val Ile Glu Arg Leu Ala Arg Ser Gln Ile
                   -60                 -55                 -50
   CAC AGC ATC CGG GAC CTC CAG CGA CTC CTG GAG ATA GAC TCC GTA GGG       256
   His Ser Ile Arg Asp Leu Gln Arg Leu Leu Glu Ile Asp Ser Val Gly
               -45                 -40                 -35
   AGT GAG GAT TCT TTG GAC ACC AGC CTG AGA GCT CAC GGG GTC CAC GCC       304
   Ser Glu Asp Ser Leu Asp Thr Ser Leu Arg Ala His Gly Val His Ala
           -30                 -25                 -20
   ACT AAG CAT GTG CCC GAG AAG CGG CCC CTG CCC ATT CGG AGG AAG AGA       352
   Thr Lys His Val Pro Glu Lys Arg Pro Leu Pro Ile Arg Arg Lys Arg
       -15                 -10                  -5
   AGC ATC GAG GAA GCT GTC CCC GCT GTC TGC AAG ACC AGG ACG GTC ATT       400
   Ser Ile Glu Glu Ala Val Pro Ala Val Cys Lys Thr Arg Thr Val Ile
     1               5                  10                  15
   TAC GAG ATT CCT CGG AGT CAG GTC GAC CCC ACG TCC GCC AAC TTC CTG       448
   Tyr Glu Ile Pro Arg Ser Gln Val Asp Pro Thr Ser Ala Asn Phe Leu
                20                  25                  30
   ATC TGG CCC CCG TGC GTG GAG GTG AAA CGC TGC ACC GGC TGC TGC AAC       496
   Ile Trp Pro Pro Cys Val Glu Val Lys Arg Cys Thr Gly Cys Cys Asn
            35                  40                  45
   ACG AGC AGT GTC AAG TGC CAG CCC TCC CGC GTC CAC CAC CGC AGC GTC       544
   Thr Ser Ser Val Lys Cys Gln Pro Ser Arg Val His His Arg Ser Val
        50                  55                  60
   AAG GTG GCC AAG GTG GAA TAC GTC AGG AAG AAG CCA AAA TTA AAA GAA       592
   Lys Val Ala Lys Val Glu Tyr Val Arg Lys Lys Pro Lys Leu Lys Glu
    65                  70                  75                  80
   GTC CAG GTG AGG TTA GAG GAG CAT TTG GAG TGC GCC TGC GCG ACC ACA       640
   Val Gln Val Arg Leu Glu Glu His Leu Glu Cys Ala Cys Ala Thr Thr
                    85                  90                  95
   AGC CTG AAT CCG GAT TAT CGG GAA GAG GAC ACG GAT GTG AGG               682
   Ser Leu Asn Pro Asp Tyr Arg Glu Glu Asp Thr Asp Val Arg
               100                 105                 110
   TGAGGATGAG CCGCAGCCCT TTCCTGGGAC ATGGATGTGG GGATCCGTCG ACCTGCAGCC     742
   AAGCTT                                                                748
(2) INFORMATION ZU SEQ ID NO: 2:
   (i) SEQUENZ CHARAKTERISTIKA:
      (A) LÄNGE: 196 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 2:
   Met Arg Thr Leu Ala Cys Leu Leu Leu Leu Gly Cys Gly Tyr Leu Ala
   -86 -85                 -80                 -75
   His Val Leu Ala Glu Glu Ala Glu Ile Pro Arg Glu Val Ile Glu Arg
   -70                 -65                 -60                 -55
   Leu Ala Arg Ser Gln Ile His Ser Ile Arg Asp Leu Gln Arg Leu Leu
                   -50                 -45                 -40
   Glu Ile Asp Ser Val Gly Ser Glu Asp Ser Leu Asp Thr Ser Leu Arg
               -35                 -30                 -25
   Ala His Gly Val His Ala Thr Lys His Val Pro Glu Lys Arg Pro Leu
           -20                 -15                 -10
   Pro Ile Arg Arg Lys Arg Ser Ile Glu Glu Ala Val Pro Ala Val Cys
        -5                  1                   5                  10
   Lys Thr Arg Thr Val Ile Tyr Glu Ile Pro Arg Ser Gln Val Asp Pro
                   15                  20                  25
   Thr Ser Ala Asn Phe Leu Ile Trp Pro Pro Cys Val Glu Val Lys Arg
               30                  35                  40
   Cys Thr Gly Cys Cys Asn Thr Ser Ser Val Lys Cys Gln Pro Ser Arg
           45                  50                  55
   Val His His Arg Ser Val Lys Val Ala Lys Val Glu Tyr Val Arg Lys
       60                  65                  70
   Lys Pro Lys Leu Lys Glu Val Gln Val Arg Leu Glu Glu His Leu Glu
   75                  80                  85                  90
   Cys Ala Cys Ala Thr Thr Ser Leu Asn Pro Asp Tyr Arg Glu Glu Asp
                   95                  100                 105
   Thr Asp Val Arg
               110
(2) INFORMATION ZU SEQ ID NO: 3:
   (i) SEQUENZ CHARAKTERISTIKA:
      (A) LANGE: 868 Basenpaare
      (B) ART: Nukleinsäure
      (C) STRANGFORM: Einzel
      (D) TOPOLOGIE: linear
   (ii) ART DES MOLEKÜLS: cDNS
   (vi) URSPRÜNGLICHE HERKUNFT:
      (A) ORGANISMUS: Homo sapiens
   (vii) UNMITTELBARE HERKUNFT:
      (B) CLON: pMVW-2 (Weich et al., 1986)
   (ix) MERKMALE:
      (A) NAME/SCHLÜSSEL: CDS
      (B) LAGE: 40..762
      (D) SONSTIGE ANGABEN: /product= "PDGF-B
         Vorlaeufersequenz"
         /note= "humanes PDGF-B Gen aus pGEM2-PDGF-B,
         flankiert von 5'-EcoRI und 3'-HindIII
         Restriktionsschnittstellen"
   (ix) MERKMALE:
      (A) NAME/SCHLÜSSEL: mat_peptide
      (B) LAGE: 283..609
      (D) SONSTIGE ANGABEN: /product= "mature PDGF-B Kette"
   (x) VERÖFFENTLICHUNGSINFORMATION:
      (A) AUTOREN: Weich, E. A.
         Sebald, W.
         Schairer, E. U.
         Hoppe, U.
      (C) ZEITSCHRIFT: FEBS Lett.
      (D) BAND: 198
      (F) SEITEN: 344-348
      (G) DATUM: 1986
   (xi) SEQUENZBESCHREIBUNG: SEQ ID NO: 3:
GAATTCGAGC TCGCCCGGGG ATCCTCTAGA GTCGACACC ATG AAT CGC TGC TGG          54
   GCG CTC TTC CTG TCT CTC TGC TGC TAC CTG CGT CTG GTC AGC GCC GAG        102
   GGG GAC CCC ATT CCC GAG GAG CTT TAT GAG ATG CTG AGT GAT CAC TCG        150
   ATC CGC TCC TTT GAT GAT CTC CAA CGC CTG CTG CAC GGA GAC CCC GGA          198
   GAG GAA GAT GGG GCC GAG TTG GAC CTG AAC ATG ACC CGC TCC CAC TCT          246
   GGA GGC GAG CTG GAG AGC TTG GCT CGT GGA AGA AGG AGC CTG GGT TCC          294
   CTG ACC ATT GCT GAG CCG GCC ATG ATC GCC GAG TGC AAG ACG CGC ACC          342
   GAG GTG TTC GAG ATC TCC CGG CGC CTC ATA GAC CGC ACC AAC GCC AAC          390
   TTC CTG GTG TGG CCG CCC TGT GTG GAG GTG CAG CGC TGC TCC GGC TGC          438
   TGC AAC AAC CGC AAC GTG CAG TGC CGC CCC ACC CAG GTG CAG CTG CGA          486
   CCT GTC CAG GTG AGA AAG ATC GAG ATT GTG CGG AAG AAG CCA ATC TTT          534
   AAG AAG GCC ACG GTG ACG CTG GAA GAC CAC CTG GCA TGC AAG TGT GAG          582
   ACA GTG GCA GCT GCA CGG CCT GTG ACC CGA AGC CCG GGG GGT TCC CAG          630
   GAG CAG CGA GCC AAA ACG CCC CAA ACT CGG GTG ACC ATT CGG ACG GTG          678
   CGA GTC CGC CGG CCC CCC AAG GGC AAG CAC CGG AAA TTC AAG CAC ACG          726
   CAT GAC AAG ACG GCA CTG AAG GAG ACC CTT GGA GCC TAGGGGCATC               772
   GGCAGGAGAG TGTGTGGGCA GGGTTATTTA ATATGGTATT TGCTGTATTG CCCCCATGGC       832
   CCAATCGATC CCGTCGACCT GCAGGCATGC AAGCTT                                  868
(2) INFORMATION ZU SEQ ID NO: 4:
   (i) SEQUENZ CHARAKTERISTIKA:
      (A) LÄNGE: 241 Aminosäuren
      (B) ART: Aminosäure
      (D) TOPOLOGIE: linear
   (ii) ART DES MOLEKÜLS: Protein
   (xi) SEQUENZBESCHREIBUNG: SEQ ID NO: 4:
(2) INFORMATION ZU SEQ ID NO: 5:
   (i) SEQUENZ CHARAKTERISTIKA:
      (A) LÄNGE: 628 Basenpaare
      (B) ART: Nukleinsäure
      (C) STRANGFORM: Einzel
      (D) TOPOLOGIE: linear
   (vi) URSPRÜNGLICHE HERKUNFT:
      (A) ORGANISMUS: Poliovirus Typ 1 (Mahoney strain)
   (vii) UNMITTELBARE HERKUNFT:
      (B) CLON: pGEM3-5'Polio (M) (4708 bp), (Sarnow, 1989)
   (ix) MERKMALE:
      (A) NAME/SCHLÜSSEL: -
      (B) LAGE: 1..628
      (D) SONSTIGE ANGABEN: /note= "abgebildet sind die ersten 628 nt der 5' nicht-translatierten Region des Poliovirus Typ 1 (Mahoney)"
   (ix) MERKMALE:
      (A) NAME/SCHLÜSSEL: -
      (B) LAGE: 610
      (D) SONSTIGE ANGABEN: /note= "nicht-authentische Sequenz auf Grund einer Basenpaarsubstitution von C nach G an der Position 610"
   (x) VERÖFFENTLICHUNGSINFORMATION:
      (A) AUTOREN: Sarnow, P.
      (C) ZEITSCHRIFT: J. Virol.
      (D) BAND: 63
      (F) SEITEN: 467-470
      (G) DATUM: 1989
   (xi) SEQUENZBESCHREIBUNG: SEQ ID NO: 5:
   TTAAAACAGC TCTGGGGTTG TACCCACCCC AGAGGCCCAC GTGGCGGCTA GTACTCCGGT    60
   ATTGCGGTAC CCTTGTACGC CTGTTTTATA CTCCCTTCCC GTAACTTAGA CGCACAAAAC   120
   CAAGTTCAAT AGAAGGGGGT ACAAACCAGT ACCACCACGA ACAAGCACTT CTGTTTCCCC   180
   GGTGATGTCG TATAGACTGC TTGCGTGGTT GAAAGCGACG GATCCGTTAT CCGCTTATGT   240
   ACTTCGAGAA GCCCAGTACC ACCTCGGAAT CTTCGATGCG TTGCGCTCAG CACTCAACCC   300
   CAGAGTGTAG CTTAGGCTGA TGAGTCTGGA CATCCCTCAC CGGTGACGGT GGTCCAGGCT   360
   GCGTTGGCGG CCTACCTATG GCTAACGCCA TGGGACGCTA GTTGTGAACA AGGTGTGAAG   420
   AGCCTATTGA GCTACATAAG AATCCTCCGG CCCCTGAATG CGGCTAATCC CAACCTCGGA   480
   GCAGGTGGTC ACAAACCAGT GATTGGCCTG TCGTAACGCG CAAGTCCGTG GCGGAACCGA   540
   CTACTTTGGG TGTCCGTGTT TCCTTTTATT TTATTGTGGC TGCTTATGGT GACAATCACA   600
   GATTGTTATG ATAAAGCGAA TTGGATTG                                      628
(2) INFORMATION ZU SEQ ID NO: 6:
   (i) SEQUENZ CHARAKTERISTIKA:
      (A) LÄNGE: 17 Basenpaare
      (B) ART: Nukleinsäure
      (C) STRANGFORM: Einzel
      (D) TOPOLOGIE: linear
   (ix) MERKMALE:
      (A) NAME/SCHLÜSSEL: -
      (B) LAGE: 1..17
      (D) SONSTIGE ANGABEN: /label= M1317MER
         /note= "synthetische DNA; M13 Sequenzierprimer (New England Biolabs GmbH), eingesetzt fuer PCR"
   (xi) SEQUENZBESCHREIBUNG: SEQ ID NO: 6:
   GTAAAACGAC GGCCAGT 17
(2) INFORMATION ZU SEQ ID NO: 7:
   (i) SEQUENZ CHARAKTERISTIKA:
      (A) LÄNGE: 24 Basenpaare
      (B) ART: Nukleinsäure
      (C) STRANGFORM: Einzel
      (D) TOPOLOGIE: linear
   (ix) MERKMALE:
      (A) NAME/SCHLÜSSEL: -
      (B) LAGE: 1..24
      (D) SONSTIGE ANGABEN: /label= M1324MER
         /note= "synthetische DNA; M13 reverser
         Sequenzierprimer (New England Biolabs GmbH),
         eingesetzt fuer PCR"
   (xi) SEQUENZBESCHREIBUNG: SEQ ID NO: 7:
   AGCGGATAAC AATTTCACAC AGGA 24
(2) INFORMATION ZU SEQ ID NO: 8:
   (i) SEQUENZ CHARAKTERISTIKA:
      (A) LÄNGE: 19 Basenpaare
      (B) ART: Nukleinsäure
      (C) STRANGFORM: Einzel
      (D) TOPOLOGIE: linear
   (ix) MERKMALE:
      (A) NAME/SCHLÜSSEL: -
      (B) LAGE: 1..19
      (D) SONSTIGE ANGABEN: /label= NCCLSA1
         /note= "synthetische DNA; synthetischer Linker zur
         Umklonierung des verkuerzten PDGF-B Vorlaeufers
         aus pMVW-2 in den Bakteriophagen M13mp19"
   (xi) SEQUENZBESCHREIBUNG: SEQ ID NO: 8:
   CATGGCCCAA TCGATCCCG 19
(2) INFORMATION ZU SEQ ID NO: 9:
   (i) SEQUENZ CHARAKTERISTIKA:
      (A) LÄNGE: 19 Basenpaare
      (B) ART: Nukleinsäure
      (C) STRANGFORM: Einzel
      (D) TOPOLOGIE: linear
   (ix) MERKMALE:
      (A) NAME/SCHLÜSSEL: -
      (B) LAGE: 1..19
      (D) SONSTIGE ANGABEN: /label= NCCLSA2
         /note- "synthetische DNA; synthetischer Linker zur
         Umklonierung des verkuerzten PDGF-B Vorlaeufers
         aus pMVW-2 in den Bakteriophagen M13mp19"
   (xi) SEQUENZBESCHREIBUNG: SEQ ID NO: 9:
   TCGACGGGAT CGATTGGGC 19
(2) INFORMATION ZU SEQ ID NO: 10:
   (i) SEQUENZ CHARAKTERISTIKA:
      (A) LÄNGE: 37 Basenpaare
      (B) ART: Nukleinsäure
      (C) STRANGFORM: Einzel
      (D) TOPOLOGIE: linear
   (ix) MERKMALE:
      (A) NAME/SCHLÜSSEL: -
      (B) LAGE: 1..37
      (D) SONSTIGE ANGABEN: /label- PDGBBCL
         /note= "synthetische DNA; Mutageneseprimer zur
         Einfuehrung einer BclI-Schnittstelle in den
         5'-Bereich des PDGF-B Vorlaeufers"
   (xi) SEQUENZBESCHREIBUNG: SEQ ID NO: 10:
   GCTTTATGAG ATGCTGAGTG ATCACTCGAT CCGCTCC 37
(2) INFORMATION ZU SEQ ID NO: 11:
   (i) SEQUENZ CHARAKTERISTIKA:
      (A) LÄNGE: 110 Basenpaare
      (B) ART: Nukleinsäure
      (C) STRANGFORM: Einzel
      (D) TOPOLOGIE: linear
   (ix) MERKMALE:
      (A) NAME/SCHLÜSSEL: -
      (B) LAGE: 1..110
      (D) SONSTIGE ANGABEN: /label= PPDGFB1
         /note= "synthetische DNA; synthetischer Linker zur
         Rekonstitution der maturen PDGF-B
         Vorlaeufersequenz"
   (xi) SEQUENZBESCHREIBUNG: SEQ ID NO: 11:
   TCGACACCAT GAATCGCTGC TGGGCGCTCT TCCTGTCTCT CTGCTGCTAC CTGCGTCTGG    60
   TCAGCGCCGA GGGGGACCCC ATTCCCGAGG AGCTTTATGA GATGCTGAGT              110
(2) INFORMATION ZU SEQ ID NO: 12:
   (i) SEQUENZ CHARAKTERISTIKA:
      (A) LÄNGE: 110 Basenpaare
      (B) ART: Nukleinsäure
      (C) STRANGFORM: Einzel
      (D) TOPOLOGIE: linear
   (ix) MERKMALE:
      (A) NAME/SCHLÜSSEL: -
      (B) LAGE: 1..110
      (D) SONSTIGE ANGABEN: /label= PPDGFB2
         /note= "synthetische DNA; synthetischer Linker zur
         Rekonstitution der maturen PDGF-B
         Vorlaeufersequenz"
   (xi) SEQUENZBESCHREIBUNG: SEQ ID NO: 12:
   GATCACTCAG CATCTCATAA AGCTCCTCGG GAATGGGGTC CCCCTCGGCG CTGACCAGAC    60
   GCAGGTAGCA GCAGAGAGAC AGGAAGAGCG CCCAGCAGCG ATTCATGGTG              110
(2) INFORMATION ZU SEQ ID NO: 13:
   (i) SEQUENZ CHARAKTERISTIKA:
      (A) LÄNGE: 30 Basenpaare
      (B) ART: Nukleinsäure
      (C) STRANGFORM: Einzel
      (D) TOPOLOGIE: linear
   (ix) MERKMALE:
      (A) NAME/SCHLÜSSEL: -
      (B) LAGE: 1..30
      (D) SONSTIGE ANGABEN: /label= 5'-POLIO1
         /note= "synthetische DNA; synthetischer
         PCR-Primer"
   (xi) SEQUENZBESCHREIBUNG: SEQ ID NO: 13:
   TTTCTGCAGA AGCTTAAAAC AGCTCTGGGG 30
(2) INFORMATION ZU SEQ ID NO: 14:
   (i) SEQUENZ CHARAKTERISTIKA:
      (A) LÄNGE: 28 Basenpaare
      (B) ART: Nukleinsäure
      (C) STRANGFORM: Einzel
      (D) TOPOLOGIE: linear
   (ix) MERKMALE:
      (A) NAME/SCHLÜSSEL: -
      (B) LAGE: 1..28
      (D) SONSTIGE ANGABEN: /label= 3'-POLIO2
         /note= "synthetische DNA; synthetischer
         PCR-Primer"
   (xi) SEQUENZBESCHREIBUNG: SEQ ID NO: 14:
   TTGCGGCCGC AATCCAATTC GCTTTATC 28
(2) INFORMATION ZU SEQ ID NO: 15:
   (i) SEQUENZ CHARAKTERISTIKA:
      (A) LANGE: 13 Basenpaare
      (B) ART: Nukleinsäure
      (C) STRANGFORM: Einzel
      (D) TOPOLOGIE: linear
   (ix) MERKMALE:
      (A) NAME/SCHLÜSSEL: -
      (B) LAGE: 1..13
      (D) SONSTIGE ANGABEN: /label= E-N-E1
         /note= "synthetische DNA; synthetischer Linker"
   (xi) SEQUENZBESCHREIBUNG: SEQ ID NO: 15:
   AATTGCGGCC GCG 13
(2) INFORMATION ZU SEQ ID NO: 16:
   (i) SEQUENZ CHARAKTERISTIKA:
      (A) LÄNGE: 13 Basenpaare
      (B) ART: Nukleinsäure
      (C) STRANGFORM: Einzel
      (D) TOPOLOGIE: linear
   (ix) MERKMALE:
      (A) NAME/SCHLÜSSEL: -
      (B) LAGE: 1..13
      (D) SONSTIGE ANGABEN: /label= E-N-E2
         /note= "synthetische DNA; synthetischer Linker"
   (xi) SEQUENZBESCHREIBUNG: SEQ ID NO: 16:
   AATTCGCGGC CGC                                                    13